# EUROPEAN PATENT SPECIFICATION

(11) **EP 2 481 337 B1**
(45) Date of publication and mention of the grant of the patent: **06.08.2014**
(21) Application number: 11759061.2
(22) Date of filing: 28.01.2011
(51) Int. Cl.: A61B 1/04

(54) **CAPSULE TYPE MEDICAL DEVICE GUIDANCE SYSTEM AND METHOD**
SYSTEM UND VERFAHREN ZUM FÜHREN EINER VERKAPSELTEN MEDIZINISCHEN VORRICHTUNG
SYSTÈME DE GUIDAGE D'UN DISPOSITIF MÉDICAL DE TYPE CAPSULE ET MÉTHODE ASSOCIÉE

(30) Priority: 26.03.2010 JP 2010073383
(43) Date of publication of application: 01.08.2012
(73) Proprietor: OLYMPUS MEDICAL SYSTEMS CORP., Tokyo 151-0072 (JP)
(72) Inventor: KAWANO, Hironao, Tokyo 151-0072 (JP)
(74) Representative: Schicker, Silvia
(86) International application number: PCT/JP2011/051755
(87) International publication number: WO 2011/118253

(56) References cited:
- EP-A1- 1 969 989
- EP-A1- 1 972 255
- WO-A1-2007/077896
- WO-A1-2007/077922

## Description

### TECHNICAL FIELD

The present invention relates to a capsule medical device guidance system for guiding a capsule medical device which has a magnetic field response unit and is introduced into a liquid in a subject and a method for guiding the capsule medical device.

### BACKGROUND ART

### 2. Description of the Related Art

Conventionally, in the field of endoscope, capsule endoscopes have appeared which include an imaging function and a wireless communication function inside a capsule-shaped casing formed into a size that can be introduced into the digestive tract of a subject such as a patient. The capsule endoscope is swallowed from a mouth of the subject, and then moves in the digestive tract by a peristaltic motion or the like. The capsule endoscope sequentially captures images inside an organ of the subject (hereinafter, the image may be referred to as an in-vivo image) and sequentially wirelessly transmits the obtained in-vivo images to a receiving device outside the subject during a period from when the capsule endoscope is introduced into the inside of the digestive tract of the subject to when the capsule endoscope is excreted to the outside of the subject.

The in-vivo images captured by the capsule endoscope are taken into an image display device via the receiving device. The image display device displays the taken in-vivo images as a still image or a moving image. A user such as a doctor or a nurse observes each in-vivo image displayed on the image display device, and examines the inside of the organ of the subject through the observation of the in-vivo images.

Further, in recent years, a guidance system which guides a capsule endoscope inside a subject by a magnetic force (hereinafter referred to as magnetic guidance) is proposed. Generally, in the guidance system, the capsule endoscope further includes a permanent magnet inside the capsule-shaped casing, and the image display device displays in real time each in-vivo image sequentially captured by the capsule endoscope inside the subject. The capsule endoscope guidance system applies a magnetic field to the capsule endoscope inside the subject and magnetically guides the capsule endoscope inside the subject to a desired position by a magnetic attracting force received from the applied magnetic field. While observing the in-vivo image displayed on the image display device, the user operates the magnetic guidance of the capsule endoscope by using an operation input unit of the system.

As the capsule endoscope, there is a capsule endoscope which sequentially captures in-vivo images while drifting in the liquid to observe the inside of the organ having a relatively large space such as a stomach or a large intestine. To intensively examine the inside of an organ having a relatively large space such as a stomach, there is a case in which the subject ingests a capsule endoscope along with a liquid to stretch the inside of the organ (specifically, to stretch folds on the inner wall of the organ) (for example, see patent literature 1). In this case, while the capsule endoscope drifts in the liquid with a predetermined posture inside the organ such as a stomach, the capsule endoscope sequentially captures images of the inside of the organ stretched by the liquid.

Patent literature 1: International Publication No. 2007/077922

Document EP 1 969 989 A1 discloses a body-insertable device system having a magnetic field generating unit and a workstation. The magnetic field generator generates a magnetic field for controlling the movement of a capsule endoscope in a liquid introduced to the digestive canal of a subject. When the magnetic field generator generates a magnetic field for attracting the capsule endoscope in the liquid, the capsule endoscope receives a magnetic force from the magnetic field generator and a buoyant force from the liquid in addition to own weight. When the buoyant force is substantially equal to the sum of the weight and the magnetic force, the capsule endoscope stays in the liquid. A magnetic field controller controls the magnetic field strength of the magnetic field generating unit, that is, the magnetic force, based on the position information of the capsule endoscope, to keep the capsule endoscope under the surface of the liquid. The magnetic field generating unit reduces the magnetic force to move the capsule endoscope upward toward the surface of the liquid according to the operation of an adjusting switch of the operating unit, and the magnetic field generating unit increases the magnetic force to move the capsule endoscope downward toward the bottom of the liquid according to the operation of the adjusting switch of the operating unit.

Document EP 1 972 255 A1 discloses an encapsulated medical device guidance system having an encapsulated medical device and a magnetic guidance unit, which generates a magnetic field for guiding the encapsulated medical device. The magnetic guidance unit comprises a guidance coil group forming an induction magnetic field generator. The group of guidance coils is a first magnetic gradient generating means, which generates a first magnetic gradient to be applied to the magnet in the capsule endoscope and pulls the endoscope in a desired direction by moving in the longitudinal, horizontal and vertical directions. In particular, one of the guidance coils eliminates the influence of gravity when pulling the capsule endoscope in a desired direction by generating a second magnetic gradient to be applied to the magnet in the capsule endoscope to cancel the force of moving down the endoscope moved by the gravity. A controller is an input unit, which instructs the advancing direction and inclination of the encapsulated endoscope by the operation of an input device such as a joystick.

### DISCLOSURE OF INVENTION

### PROBLEM TO BE SOLVED BY THE INVENTION

Even with the abovementioned conventional technology, all the time the user wishes the capsule endoscope to keep in a static state; he/she needs to continue directly adjusting the operation input unit while seeing the screen so that a magnetic attracting force is generated to keep the capsule endoscope in the static state. Moreover, with the conventional technology, even when a magnetic field is generated in accordance with an operation instruction by the user, a part of the magnetic attracting force of the generated magnetic field may be consumed to counterbalance the gravity force and the buoyancy of the capsule endoscope and the capsule endoscope cannot move in a user's intended direction; therefore, the operational feeling of the user does not match the movement of the capsule endoscope and the operability is low.

The present invention has been achieved to solve the above problems with the conventional technology and it is an object of the present invention to provide a capsule medical device guidance system with increased operability of a magnetic guidance system by the user and a method for guiding the capsule medical device.

### MEANS FOR SOLVING PROBLEM

To solve the above problems and achieve the above object, it is provided a capsule medical device guidance system according to claim 1, which is a capsule medical device guidance system for guiding a capsule medical device which has a magnetic field response unit and is introduced into a liquid in a subject. The capsule medical device guidance system includes a magnetic field generator that applies a magnetic field having a magnetic field gradient to the magnetic field response unit and guides the capsule medical device a first setting unit that sets a magnetic field gradient of a magnetic field for generating a vertical magnetic attracting force which makes a resultant force of a buoyancy of the capsule medical device and a gravity force and the vertical magnetic attracting force of the capsule medical device in the liquid substantially zero as a first magnetic field gradient an operation input unit to which guidance instruction information for guiding the capsule medical device by a magnetic field is inputted a second setting unit that obtains a magnetic field gradient of a magnetic field for generating a magnetic attracting force corresponding to the guidance instruction information inputted by the operation input unit and sets the obtained magnetic field gradient of the magnetic field as a second magnetic field gradient; and a control unit that controls the magnetic field generator to apply the magnetic field having the first magnetic field gradient set by the first setting unit when the guidance instruction information is not inputted by the operation input unit and controls the magnetic field generator to apply a magnetic field having the first magnetic field gradient set by the first setting unit added with the second magnetic field gradient set by the second setting unit when the guidance instruction information is inputted by the operation input unit.

In the capsule medical device guidance system according to the present invention, the first setting unit sets a magnetic field gradient of a magnetic field for generating a vertical magnetic attracting force which makes a resultant force of the buoyancy of the capsule medical device and the gravity force and the vertical magnetic attracting force of the capsule medical device in the liquid substantially zero on the basis of a physical parameter of the capsule medical device and a physical parameter of the liquid.

The capsule medical device guidance system according to the present invention further includes a movement detector that detects a vertical movement of the capsule medical device. The first setting unit sets the first magnetic field gradient on the basis of a detection result of the movement detector.

The capsule medical device guidance system according to the present invention further includes a vertical direction component input unit that inputs guidance instruction information for guiding the capsule medical device toward a direction including a vertical direction component into the operation input unit. The movement detector sets the first magnetic field gradient on the basis of a detection result of the vertical movement of the capsule medical device only when the guidance instruction information is not inputted by the vertical direction component input unit.

The capsule medical device guidance system according to the present invention further includes a position detector that detects a position of the capsule medical device. The first setting unit sets the first magnetic field gradient on the basis of a detection result of the position detector and magnetic field distribution information obtained in advance.

The capsule medical device guidance system according to the present invention further includes a first adjustment unit that inputs instruction information for instructing a gradient adjustment of the first magnetic field gradient into the first setting unit. The first setting unit sets the first magnetic field gradient on the basis of the instruction information inputted by the first adjustment unit.

In the capsule medical device guidance system according to the present invention, the physical parameter of the capsule medical device includes a volume, a mass, and a magnetic moment of the capsule medical device. The physical parameter of the liquid includes a density of the liquid.

The capsule medical device guidance system according to the present invention further includes a physical parameter related information input unit that inputs information related to at least one of the volume, the mass, and the magnetic moment of the capsule medical device and the density of the liquid.

The capsule medical device guidance system according to the present invention further includes a vertical direction component input unit that inputs guidance instruction information for guiding the capsule medical device toward a direction including a vertical direction component into the operation input unit.

The capsule medical device guidance system according to the present invention further includes a second adjustment unit that inputs instruction information for instructing a gradient adjustment of the second magnetic field gradient into the second setting unit. The second setting unit sets the second magnetic field gradient on the basis of the instruction information inputted by the second adjustment unit.

In the capsule medical device guidance system according to the present invention, the second adjustment unit inputs instruction information for instructing a gradient adjustment of the second magnetic field gradient into the second setting unit. The second setting unit defines a range of a magnetic field gradient, whose center value is the first magnetic field gradient set by the first setting unit and which has a range width instructed by the instruction information inputted from the second adjustment unit, as an allowable setting range of the second magnetic field gradient and sets the second magnetic field gradient so that the second magnetic field gradient is included in the allowable setting range of the second magnetic field gradient.

In the capsule medical device guidance system according to the present invention, the guidance instruction information includes upper boundary arrangement instruction information for arranging the capsule medical device on an upper boundary of the liquid and lower boundary arrangement instruction information for arranging the capsule medical device on a lower boundary of the liquid. When the upper boundary arrangement instruction information is inputted as the guidance instruction information, the second setting unit sets a magnetic field gradient of a magnetic field for generating a vertically upward magnetic attracting force by a maximum force within a predetermined allowable generation range of magnetic attracting force as the second magnetic field gradient, and when the lower boundary arrangement instruction information is inputted as the guidance instruction information, the second setting unit sets a magnetic field gradient of a magnetic field for generating a vertically downward magnetic attracting force by a maximum force within the allowable generation range of magnetic attracting force as the second magnetic field gradient.

A method for guiding a capsule medical device according to the present invention is a method for guiding a capsule medical device which has a magnetic field response unit and is introduced into a liquid in a subject. The method includes a first setting step of setting a magnetic field gradient of a magnetic field for generating a vertical magnetic attracting force which makes a resultant force of a buoyancy of the capsule medical device and a gravity force and the vertical magnetic attracting force of the capsule medical device in the liquid substantially zero as a first magnetic field gradient; an operation receiving step of receiving guidance instruction information for guiding the capsule medical device by a magnetic field; a second setting step of obtaining a magnetic field gradient of a magnetic field for generating a magnetic attracting force corresponding to the guidance instruction information that is received at the operation receiving step and setting the obtained magnetic field gradient of the magnetic field as a second magnetic field gradient; and a control step of controlling a magnetic field generator such that the magnetic field having the first magnetic field gradient that is set at the first setting step is applied when the guidance instruction information is not received at the operation receiving step and controlling the magnetic field generator such that a magnetic field is applied that has the first magnetic field gradient that is set at the first setting step added with the second magnetic field gradient that is set at the second setting step when the guidance instruction information is received.

In the method for guiding a capsule medical device according to the present invention, the first setting step, at which the magnetic field gradient is set as the first magnetic field gradient, includes obtaining a magnetic field gradient of a magnetic field for generating vertical magnetic attracting force which makes a resultant force of a buoyancy of the capsule medical device and a gravity force and the vertical magnetic attracting force of the capsule medical device in the liquid substantially zero on the basis of a physical parameter of the capsule medical device and a physical parameter of the liquid.

### EFFECT OF THE INVENTION

According to the present invention, a magnetic field gradient of a magnetic field for generating a vertical magnetic attracting force which makes a resultant force of a buoyancy of a capsule medical device and a gravity force and the vertical magnetic attracting force of the capsule medical device in a liquid substantially zero is set as a first magnetic field gradient; a magnetic field gradient of a magnetic field for generating a magnetic attracting force corresponding to guidance instruction information is obtained and the obtained magnetic field gradient of the magnetic field is set as a second magnetic field gradient; and when the guidance instruction information is not inputted, a magnetic field generator is controlled such that it applies the magnetic field having the first magnetic field gradient and when the guidance instruction information is inputted, the magnetic field generator is controlled such that it applies a magnetic field having the first magnetic field gradient added with the second magnetic field gradient. With this configuration, the capsule medical device can keep in a static state without a direct operation by the user and, moreover, almost all the magnetic attracting force by a magnetic field that is applied according to the guidance instruction information is consumed to guide the capsule medical device; therefore, the operational feeling of the user matches the movement of the capsule medical device and the operability of the magnetic guidance system by the user is increased.

### BRIEF DESCRIPTION OF DRAWINGS

[Fig. 1] FIG. 1 is a schematic diagram illustrating an entire configuration of a capsule medical device guidance system according to a first embodiment.
[Fig. 2] FIG. 2 is a cross-sectional schematic diagram illustrating a configuration example of the capsule endoscope illustrated in FIG. 1.
[Fig. 3] FIG. 3 is a conceptual diagram explaining a state of the capsule endoscope in a liquid introduced into a subject.
[Fig. 4] FIG. 4 is a diagram explaining a magnetization direction of a permanent magnet in the capsule endoscope.
[Fig. 5] FIG. 5 is a conceptual diagram explaining an example of a posture of the capsule endoscope in the liquid introduced into the subject.
[Fig. 6] FIG. 6 is a diagram illustrating an example of an image displayed on a display screen of a display unit illustrated in FIG. 1.
[Fig. 7] FIG. 7 is a diagram explaining a uniform gradient magnetic field generated by a magnetic field generator illustrated in FIG. 1.
[Fig. 8] FIG. 8 is a flowchart illustrating a processing procedure when a magnetic guidance is performed by a control unit illustrated in FIG. 1.
[Fig. 9] FIG. 9 is a conceptual diagram explaining movements of the capsule endoscope in the liquid introduced into the subject.
[Fig. 10] FIG. 10 is a conceptual diagram explaining movements of the capsule endoscope in the liquid introduced into the subject.
[Fig. 11] FIG. 11 is a diagram of an example of the operation input unit illustrated in FIG. 1.
[Fig. 12] FIG. 12 is a diagram explaining the magnetic guidance of the capsule medical device which can be operated by the operation input unit illustrated in FIG. 1.
[Fig. 13] FIG. 13 is a diagram illustrating a menu screen displayed on the display unit illustrated in FIG. 1.
[Fig. 14] FIG. 14 is a diagram of an example of the operation input unit illustrated in FIG. 1.
[Fig. 15] FIG. 15 is a diagram explaining another example of the magnetic guidance of the capsule medical device which can be operated by the operation input unit illustrated in FIG. 1.
[Fig. 16] FIG. 16 is a schematic diagram illustrating an entire configuration of a capsule medical device guidance system according to a second embodiment.
[Fig. 17] FIG. 17 is a cross-sectional schematic diagram illustrating a configuration example of the capsule endoscope illustrated in FIG. 16.
[Fig. 18] FIG. 18 is a flowchart illustrating a processing procedure when a magnetic guidance is performed by a control unit illustrated in FIG. 16.
[Fig. 19] FIG. 19 is a flowchart illustrating another processing procedure when a magnetic guidance is performed by a control unit illustrated in FIG. 16.
[Fig. 20] FIG. 20 is a schematic diagram illustrating an entire configuration of a capsule medical device guidance system according to a first modified example of the second embodiment.
[Fig. 21] FIG. 21 is a flowchart illustrating a processing procedure when a magnetic guidance is performed by a control unit illustrated in FIG. 20.
[Fig. 22] FIG. 22 is a schematic diagram illustrating an entire configuration of a capsule medical device guidance system according to a second modified example of the second embodiment.
[Fig. 23] FIG. 23 is a cross-sectional schematic diagram illustrating a configuration example of the capsule endoscope illustrated in FIG. 22.
[Fig. 24] FIG. 24 is a schematic diagram illustrating an entire configuration of a capsule medical device guidance system according to a third modified example of the second embodiment.
[Fig. 25] FIG. 25 is a cross-sectional schematic diagram illustrating a configuration example of the capsule endoscope illustrated in FIG. 24.
[Fig. 26] FIG. 26 is a schematic diagram illustrating an entire configuration of a capsule medical device guidance system according to a fourth modified example of the second embodiment.
[Fig. 27] FIG. 27 is a schematic diagram illustrating an entire configuration of a capsule medical device guidance system according to a fifth modified example of the second embodiment.
[Fig. 28] FIG. 28 is a cross-sectional schematic diagram illustrating a configuration example of the capsule endoscope illustrated in FIG. 27.
[Fig. 29] FIG. 29 is a schematic diagram illustrating an entire configuration of a capsule medical device guidance system according to a third embodiment.
[Fig. 30] FIG. 30 is a diagram of an example of the operation input unit illustrated in FIG. 29 and a first adjustment unit.
[Fig. 32] FIG. 31 is a schematic diagram illustrating an entire configuration of a capsule medical device guidance system according to a fourth embodiment.

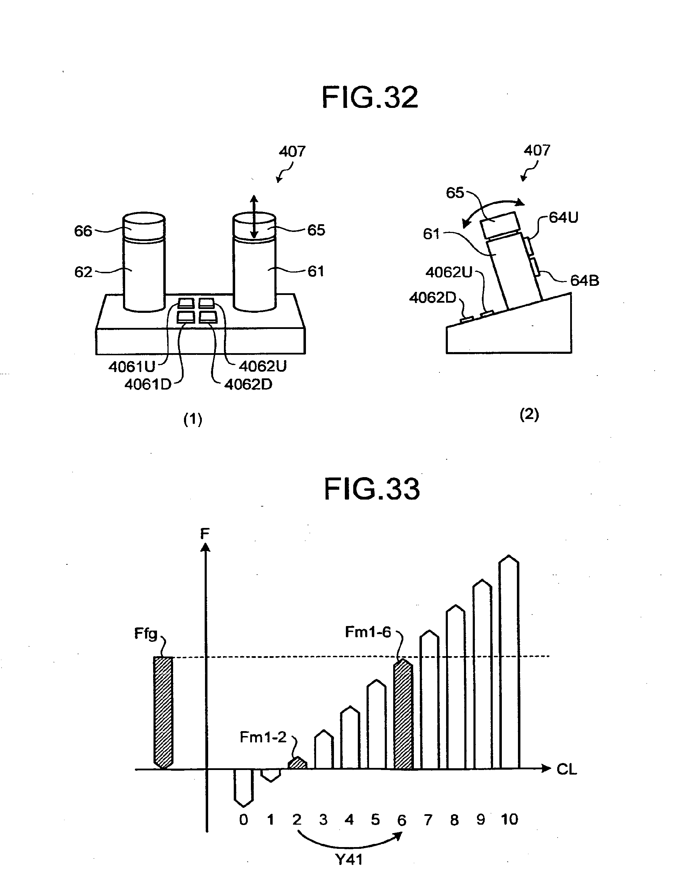

[Fig. 32] FIG. 32 is a diagram of an example of the operation input device according to the fourth embodiment.
[Fig. 33] FIG. 33 is a diagram explaining an adjustment of a first magnetic field gradient according to the fourth embodiment.

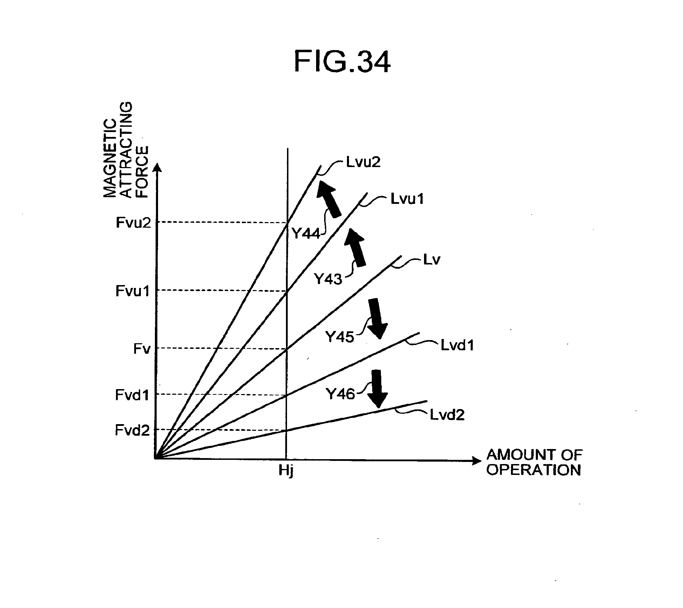

[Fig. 34] FIG. 34 is a diagram illustrating a relationship between an amount of operation of a joystick and a generated magnetic attracting force.

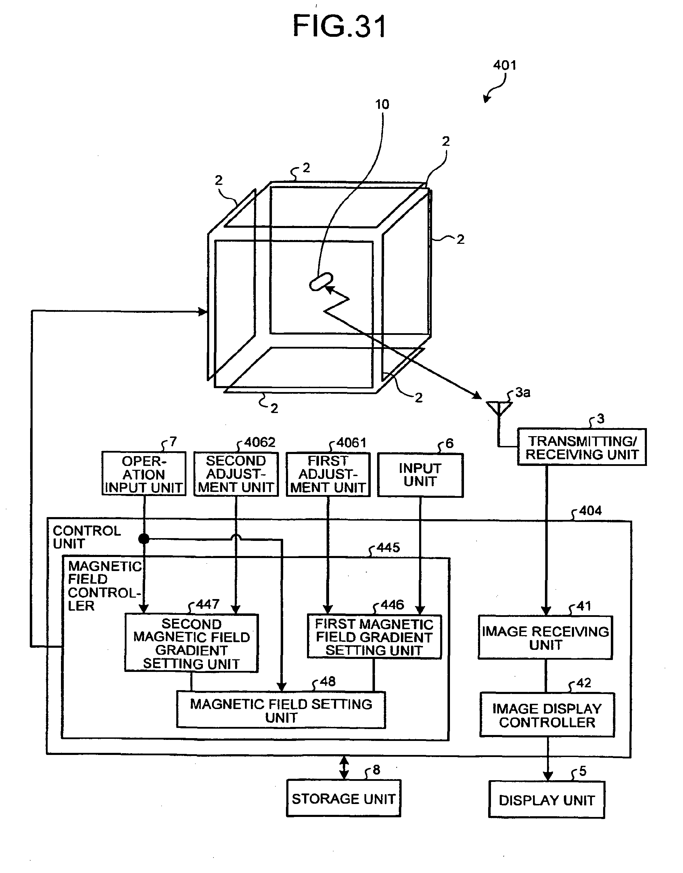

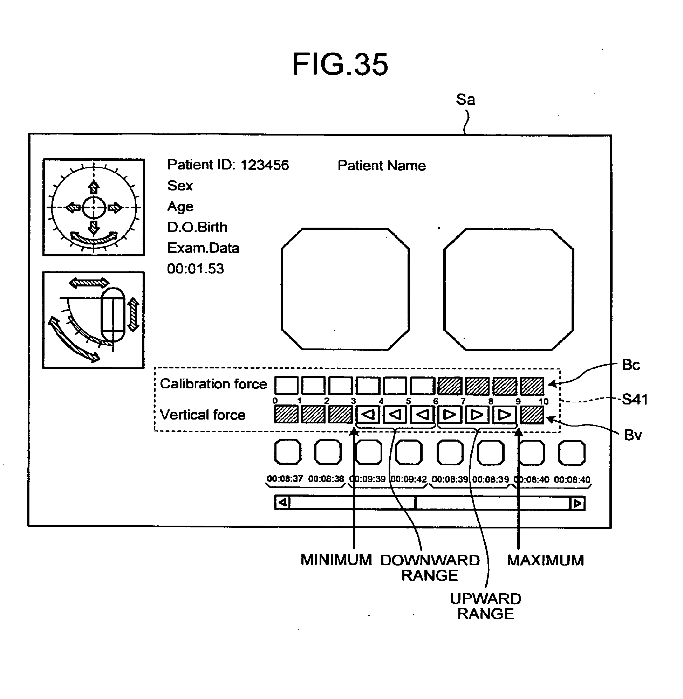

[Fig. 35] FIG. 35 is a diagram illustrating an example of a menu screen displayed on a display unit illustrated in FIG. 31.

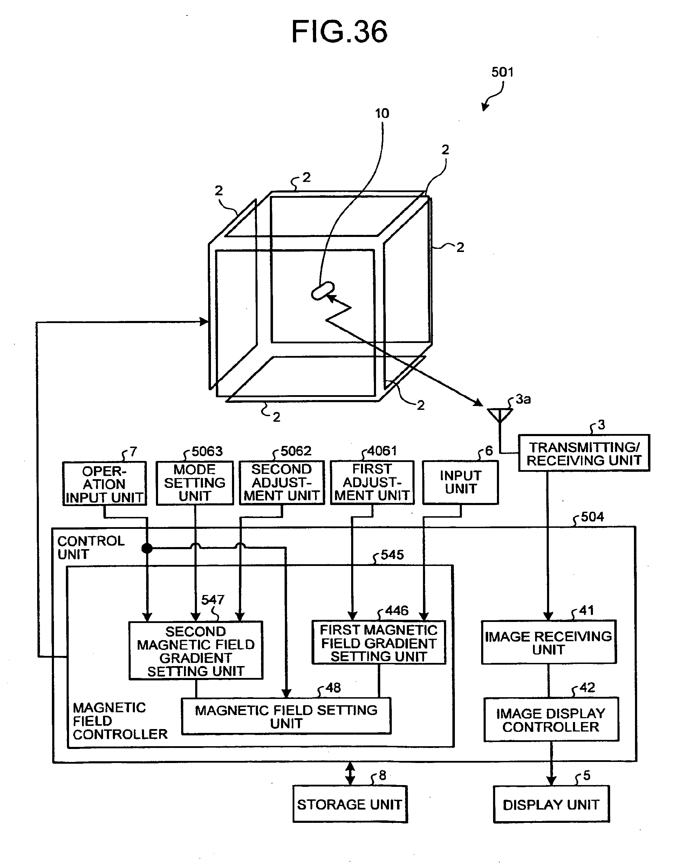

[Fig. 36] FIG. 36 is a schematic diagram illustrating an entire configuration of a capsule medical device guidance system according to a fifth embodiment.

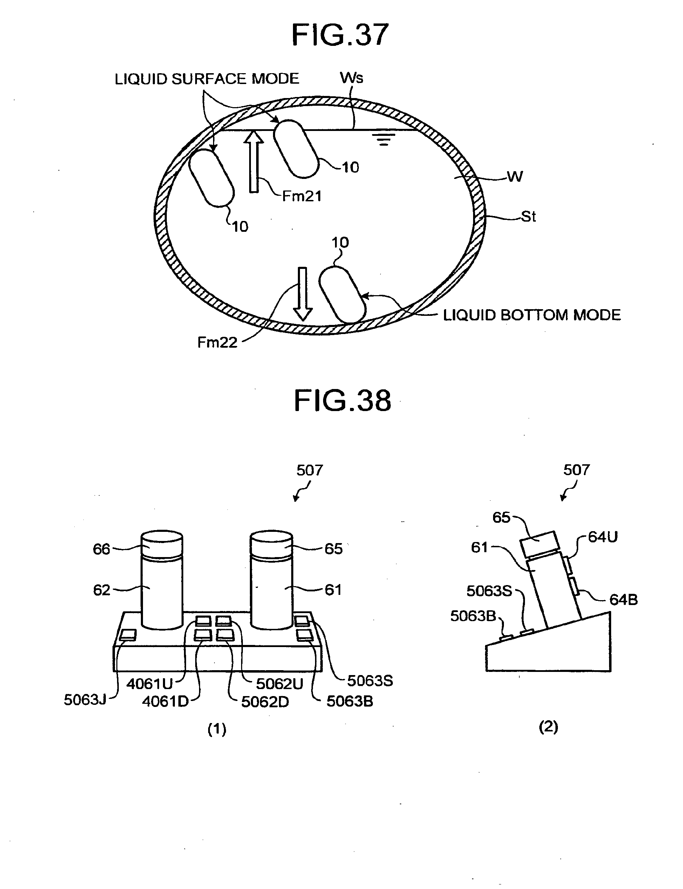

[Fig. 37] FIG. 37 is a diagram explaining a liquid surface mode and a liquid bottom mode according to the fifth embodiment.

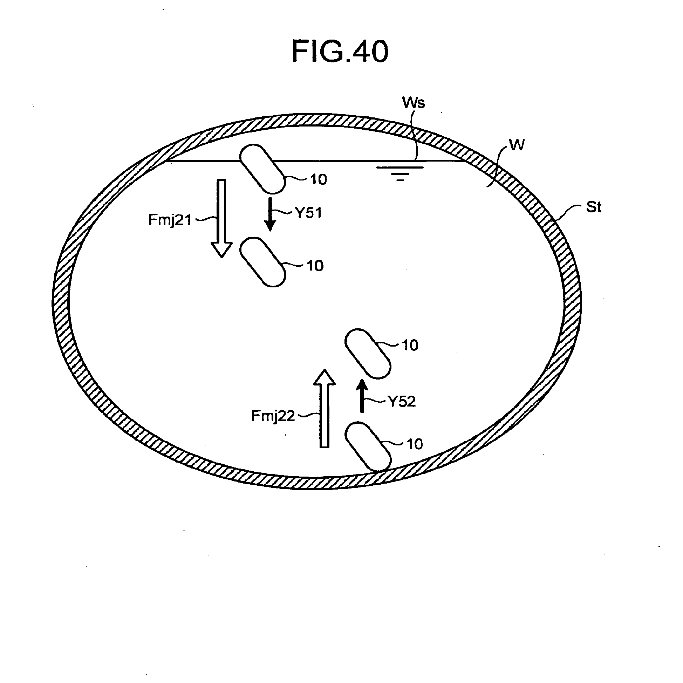

[Fig. 38] FIG. 40 is a diagram of an example of the operation input device according to the fifth embodiment.

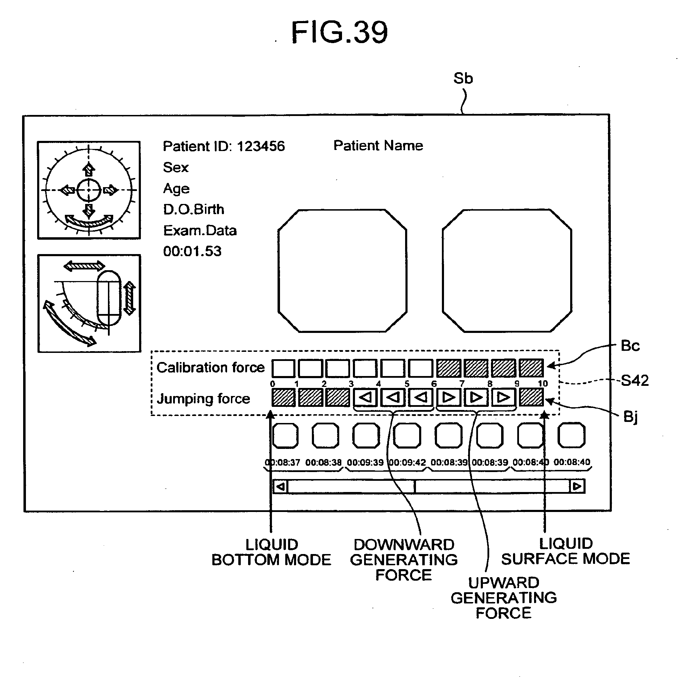

[Fig. 39] FIG. 39 is a diagram illustrating an example of a menu screen displayed on a display unit illustrated in FIG. 36.
[Fig. 40] FIG. 40 is a diagram explaining a jumping mode according to the fifth embodiment.

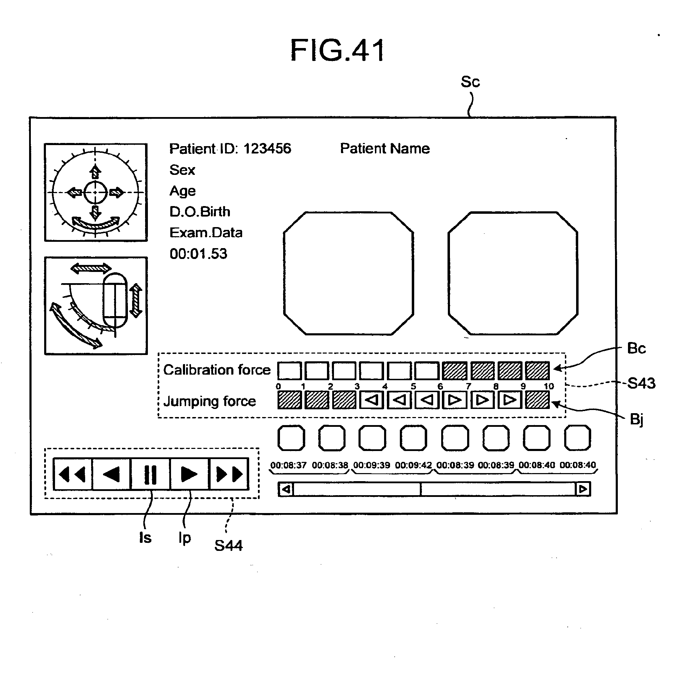

[Fig. 41] FIG. 41 is a diagram illustrating an example of a menu screen displayed on the display unit illustrated in FIG. 36.

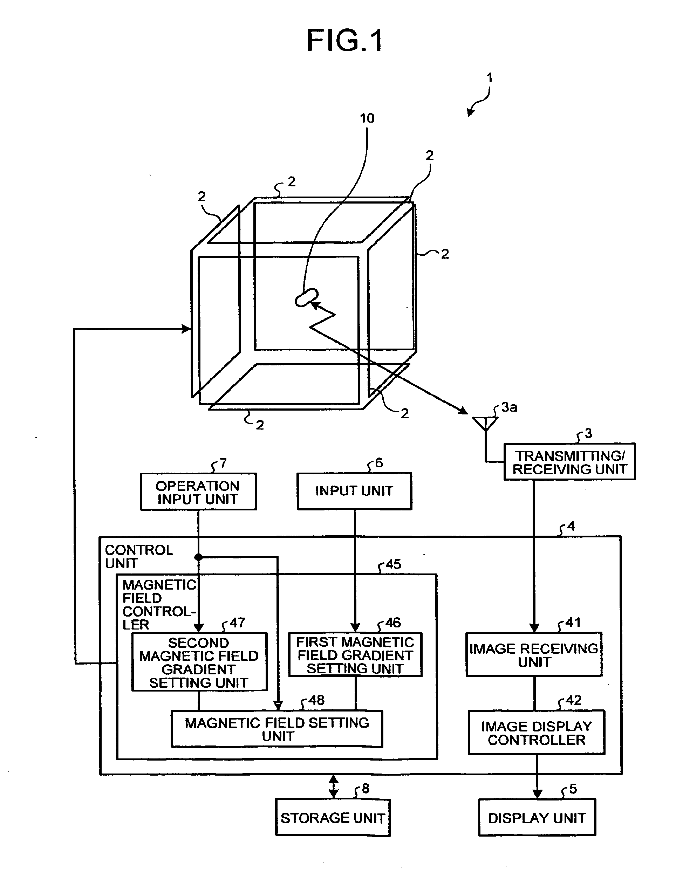

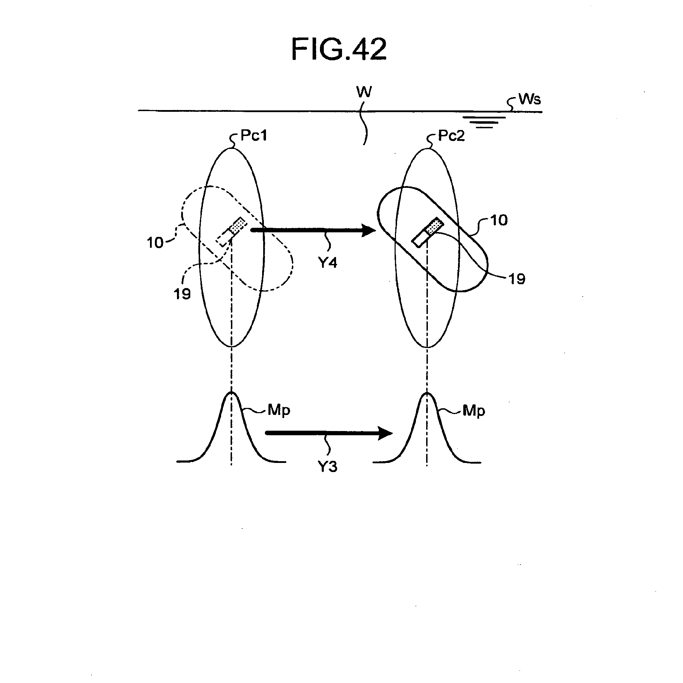

[Fig. 42] FIG. 42 is a diagram explaining a peak magnetic field generated by a magnetic field generator illustrated in FIG. 1.

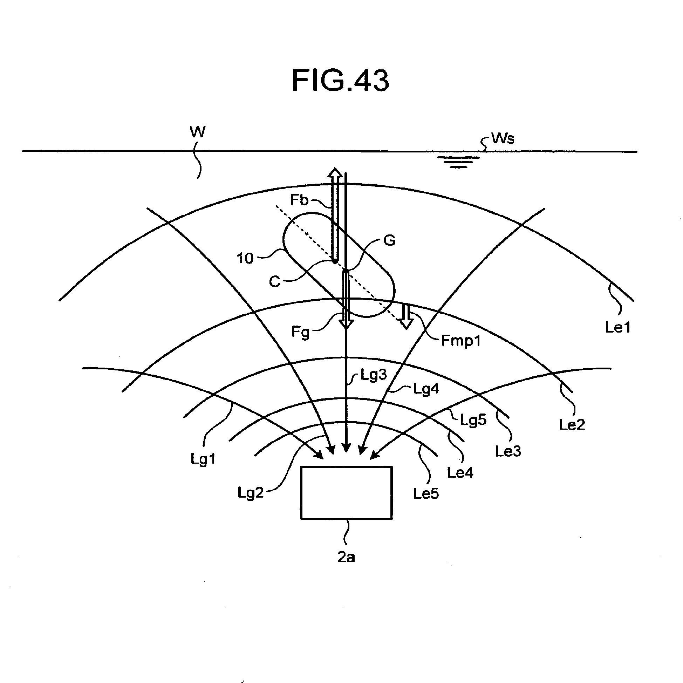

[Fig. 43] FIG. 43 is a conceptual diagram explaining a case in which a capsule endoscope is guided by using the peak magnetic field.

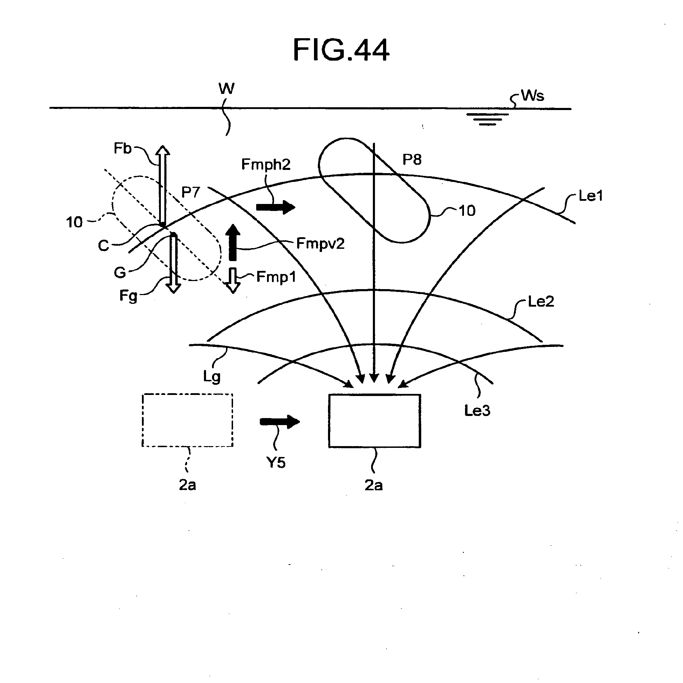

[Fig. 44] FIG. 44 is a conceptual diagram explaining a case in which a capsule endoscope is guided by using the peak magnetic field.

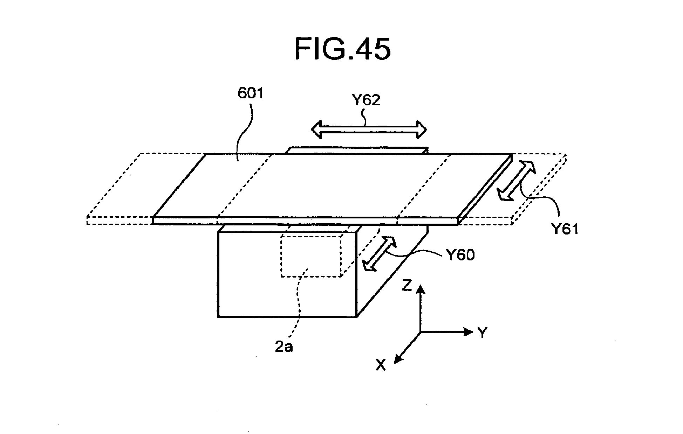

[Fig. 45] FIG. 45 is a schematic diagram illustrating an example of movement states of a table of a bed included in the magnetic field generator illustrated in FIG. 1 and the magnetic field generator.

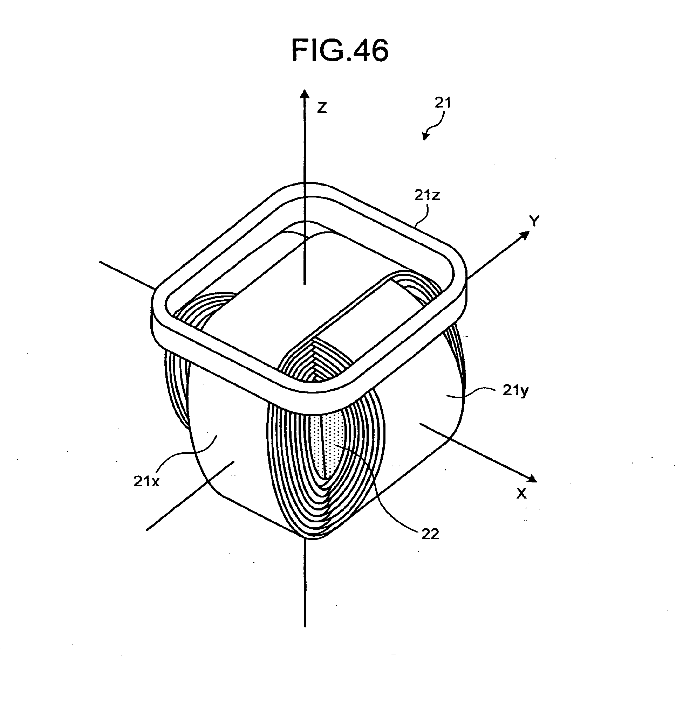

[Fig. 46] FIG. 46 is a schematic diagram illustrating an example of the magnetic field generator illustrated in FIG. 1.

### BEST MODE(S) FOR CARRYING OUT THE INVENTION

Hereinafter, capsule medical device guidance systems according to embodiments of the present invention will be described by using an example of a capsule medical device system that uses a capsule endoscope which is orally introduced into a subject and drifts in a liquid stored in a stomach of a subject. However, not limited to this, various capsule medical devices can be used, such as a capsule endoscope that moves through a lumen from an esophagus to an anus of a subject and a capsule endoscope that is introduced from an anus along with an isotonic solution. The embodiments do not limit the present invention. In the drawings, the same components are given the same reference numerals.

### (First Embodiment)

First, a first embodiment will be described. FIG. 1 is a schematic diagram illustrating an entire configuration of the capsule medical device guidance system according to a first embodiment of the present invention. As illustrated in FIG. 1, a capsule medical device guidance system 1 according to the first embodiment includes a capsule endoscope 10 which is a capsule medical device that is swallowed from a mouth of a subject, introduced into a body cavity in the subject, and communicates with an external device; a magnetic field generator 2 which is provided around the subject and can generate a three-dimensional magnetic field; a transmitting/receiving unit 3 which performs wireless communication with the capsule endoscope 10, receives a wireless signal including an image captured by the capsule endoscope 10, and transmits an operation signal to the capsule endoscope 10; a control unit 4 which controls each component of the capsule medical device guidance system 1; a display unit 5 which outputs and displays the image captured by the capsule endoscope 10; an input unit 6 which inputs instruction information for instructing various operations in the capsule medical device guidance system 1; an operation input unit 7 to which guidance instruction information for magnetically guiding the capsule endoscope 10 is inputted; and a storage unit 8 which stores image information captured by the capsule endoscope 10 and the like.

The capsule endoscope 10 is a capsule type medical device that obtains in-vivo images of the subject. The capsule endoscope 10 includes an image capturing function and a wireless communication function. The capsule endoscope 10 is introduced inside an organ of the subject along with a predetermined liquid by an oral intake or the like, and then, moves inside the digestive tract, and finally, the capsule endoscope 10 is excreted to the outside of the subject. The capsule endoscope 10 sequentially captures in-vivo images in the subject and sequentially transmits the obtained in-vivo images to the external transmitting/receiving unit 3. The capsule endoscope 10 contains a magnetic body such as a permanent magnet. The capsule endoscope 10 drifts in the liquid introduced inside of an organ (for example, stomach) of the subject, and the capsule endoscope 10 is magnetically guided by the external magnetic field generator 2.

The magnetic field generator 2 magnetically guides the capsule endoscope 10 inside the subject. The magnetic field generator 2 is realized by using, for example, a plurality of coils, and generates guidance magnetic field by using electric power supplied by a power supply unit not illustrated in FIG. 1. The magnetic field generator 2 applies the generated guidance magnetic field to the magnetic body inside the capsule endoscope 10 to magnetically capture the capsule endoscope 10 by an action of the guidance magnetic field. The magnetic field generator 2 controls three-dimensional posture of the capsule endoscope 10 inside the subject by changing a magnetic field direction of the guidance magnetic field acting on the capsule endoscope 10 inside the subject.

The transmitting/receiving unit 3 includes a plurality of antennas 3a, and receives the in-vivo images of the subject from the capsule endoscope 10 through the antennas 3a. The transmitting/receiving unit 3 sequentially receives wireless signals from the capsule endoscope 10 through the antennas 3a. The transmitting/receiving unit 3 selects an antenna which has the highest received electric-field strength from the plurality of antennas 3a, and performs demodulation processing or the like on the wireless signals received from the capsule endoscope 10 through the selected antenna. Thereby, the transmitting/receiving unit 3 extracts image data captured by the capsule endoscope 10, that is to say, in-vivo image data of the subject, from the wireless signals. The transmitting/receiving unit 3 transmits an image signal including the extracted in-vivo image data to the control unit 4.

The control unit 4 controls each operation of the magnetic field generator 2, the transmitting/receiving unit 3, the display unit 5, and the storage unit 8, and controls input and output of signals between these components. The control unit 4 controls the storage unit 8 to store an in-vivo image group of the subject obtained from the transmitting/receiving unit 3. The control unit 4 includes an image receiving unit 41 that sequentially obtains the in-vivo images sequentially received by the transmitting/receiving unit 3, an image display controller 42 that displays the in-vivo images sequentially received by the transmitting/receiving unit 3 in real time on the display unit 5, and a magnetic field controller 45 that controls the magnetic field generator 2 to guide the capsule endoscope 10. The magnetic field controller 45 controls the amount of current supplied to the magnetic field generator 2, and controls the magnetic field generator 2 to generate a guidance magnetic field necessary for the magnetic guidance of the capsule endoscope 10 in accordance with a magnetic guidance direction and a magnetic guidance position based on the guidance instruction information.

The magnetic field controller 45 includes a first magnetic field gradient setting unit 46, a second magnetic field gradient setting unit 44, and a magnetic field setting unit 48. The first magnetic field gradient setting unit 46 obtains a magnetic field gradient of a magnetic field that generates a vertical magnetic attracting force which makes a resultant force of the buoyancy of the capsule endoscope 10 and the gravity force and the vertical magnetic attracting force of the capsule endoscope 10 in the liquid substantially zero, based on a physical parameter of the capsule endoscope 10 and a physical parameter of the liquid, and sets the obtained magnetic field gradient as a first magnetic field gradient. The second magnetic field gradient setting unit 47 obtains a magnetic field gradient of a magnetic field that generates a magnetic attracting force corresponding to guidance instruction information for changing the position of the capsule endoscope 10, which is inputted from the operation input unit 7 described later, and sets the obtained magnetic field gradient as a second magnetic field gradient. When the guidance instruction information for changing the position of the capsule endoscope 10 is not inputted from the operation input unit 7, the magnetic field setting unit 48 controls the magnetic field generator 2 to apply a magnetic field having the first magnetic field gradient set by the first magnetic field gradient setting unit 46 and orient the magnetic field in a direction corresponding to the guidance instruction information for changing the position of the capsule endoscope 10, which is inputted from the operation input unit 7 described later. When the guidance instruction information for changing the position of the capsule endoscope 10 is inputted from the operation input unit 7, the magnetic field setting unit 48 controls the magnetic field generator 2 to apply a magnetic field having a magnetic field gradient obtained by adding the second magnetic field gradient set by the second magnetic field gradient setting unit 47 to the first magnetic field gradient set by the first magnetic field gradient setting unit 46 and orient the magnetic field in a direction corresponding to the guidance instruction information for changing the position of the capsule endoscope 10, which is inputted from the operation input unit 7 described later. The physical parameter of the capsule endoscope 10 includes the volume, the mass, and the magnetic moment of the capsule endoscope 10. The physical parameter of the liquid includes the density of the liquid.

The display unit 5 is realized by using various types of display such as a liquid crystal display, and displays various information which is instructed to display by the control unit 4. Specifically, the display unit 5 displays, for example, the in-vivo image group captured by the capsule endoscope 10 on the basis of the control of the image display controller 42 in the control unit 4. Further, the display unit 5 displays a reduced image of an in-vivo image selected or marked from the in-vivo image group by an input operation of the input unit 6, patient information and examination information of the subject, and the like.

The input unit 6 is realized by input devices such as a keyboard and a mouse, and the input unit 6 inputs various information into the control unit 4 according to an input operation by a user such as a doctor. Examples of the various information inputted from the input unit 6 to the control unit 4 include instruction information for instructing the control unit 4, the patient information of the subject, and the examination information of the subject. The patient information of the subject is identification information for identifying the subject, and includes, for example, patient name, patient ID, birth date, sex, and age of the subject. The examination information of the subject is identification information for identifying an examination in which the capsule endoscope 10 is introduced into the digestive tract of the subject and the inside of the digestive tract is observed, and for example, the examination information is examination ID, examination date, and the like.

The guidance instruction information for magnetically guiding the capsule endoscope 10 is inputted into the operation input unit 7. The operation input unit 7 inputs the guidance instruction information for magnetically guiding the capsule endoscope 10, such as the posture and the position of the capsule endoscope 10, which is the target of magnetic guidance operation, into the control unit 4. The operation input unit 7 has a configuration including a joystick, various buttons, and various switches. When a user operates the joystick and the like, the operation input unit 7 inputs the guidance instruction information into the control unit 4.

The storage unit 8 is realized by using a storage medium such as flash memory or a hard disk which rewritably stores data. The storage unit 8 stores various information instructed to be stored by the control unit 4, and transmits information instructed to be read from the stored various information by the control unit 4 to the control unit 4. The various information stored by the storage unit 8 includes, for example, each image data in the in-vivo image group of the subject captured by the capsule endoscope 10, data of the in-vivo image selected from each in-vivo image displayed by the display unit 5 by an input operation of the input unit 6, input information by the input unit 6, such as the patient information of the subject, and the like.

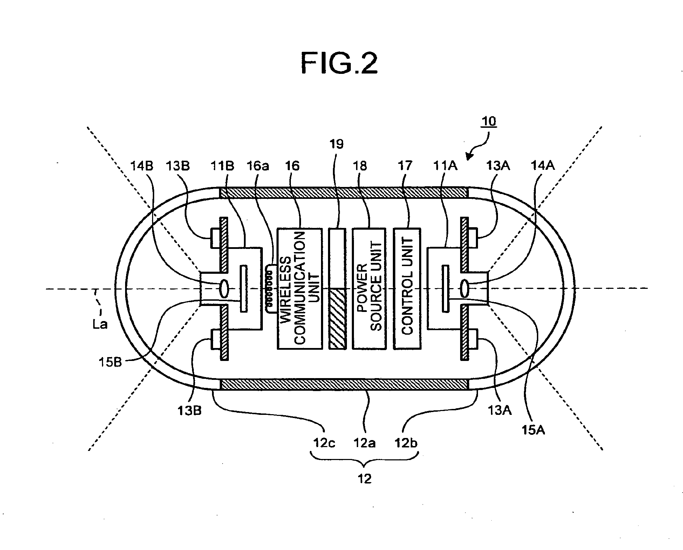

Next, the capsule endoscope 10 will be described. FIG. 2 is a cross-sectional schematic diagram illustrating a configuration example of the capsule endoscope illustrated in FIG. 1. As illustrated in FIG. 2, the capsule endoscope 10 includes a capsule-shaped casing 12 that is an outer covering formed in a size which can be easily introduced into the inside of an organ of the subject and imaging units 11A and 11B that respectively capture images of the subject in the image capturing directions different from each other. The capsule endoscope 10 also includes a wireless communication unit 16 that wirelessly transmits images captured by the imaging units 11A and 11B to the outside, a control unit 17 that controls each component of the capsule endoscope 10, and a power source unit 18 that supplies electric power to each component of the capsule endoscope 10. Further, the capsule endoscope 10 includes a permanent magnet 19 that enables the magnetic guidance by the magnetic field generator 2.

The capsule-shaped casing 12 is an outer casing formed in a size capable of being introduced into the inside of an organ of the subject, and the capsule-shaped casing 12 is realized by closing both opening ends of a tubular casing 12a by dome-shaped casings 12b and 12c. The dome-shaped casings 12b and 12c are dome-shaped optical members transparent to light of a predetermined wavelength band such as visible light. The tubular casing 12a is a colored casing that is substantially opaque to visible light. As illustrated in FIG. 2, the capsule-shaped casing 12 formed by the tubular casing 12a and the dome-shaped casings 12b and 12c liquid-tightly contains the imaging units 11A and 11B, the wireless communication unit 16, the control unit 17, the power source unit 18, and the permanent magnet 19.

The imaging units 11A and 11B respectively capture images in the image capturing directions different from each other. Specifically, the imaging unit 11A has an illumination unit 13A such as an LED, an optical system 14A such as a condenser lens, and an imaging element 15A such as a CMOS image sensor or a CCD. The illumination unit 13A emits illumination light such as white light to an imaging view field of the imaging element 15A and illuminates the subject in the imaging view field through the dome-shaped casing 12b. The optical system 14A collects reflected light from the imaging view field on an imaging surface of the imaging element 15A and forms a subject image of the imaging view field on the imaging surface of the imaging element 15A. The imaging element 15A receives the reflected light from the imaging view field via the imaging surface and photoelectrically converts the received light signal to capture the subject image of the imaging view field, which is an in-vivo image of the subject. In the same manner as the imaging unit 11A, the imaging unit 11B has an illumination unit 13B such as an LED, an optical system 14B such as a condenser lens, and an imaging element 15B such as a CMOS image sensor or a CCD. As illustrated in FIG. 2, when the capsule endoscope 10 is a two-lens capsule medical device that captures images in the front and the rear of the device in the long axis La direction, the optical axes of the imaging units 11A and 11B are substantially in parallel with the long axis La or substantially correspond to the long axis La which is the central axis in the longitudinal direction of the capsule-shaped casing 12. The directions of the imaging view fields of the imaging units 11A and 11B, in other words, the image capturing directions of the imaging units 11A and 11B, are opposite to each other.

The wireless communication unit 16 includes an antenna 16a and sequentially wirelessly transmits images captured by the above-described imaging units 11A and 11B to the outside via the antenna 16a. Specifically, the wireless communication unit 16 obtains an image signal of an in-vivo image of the subject captured by the imaging unit 11A or the imaging unit 11B from the control unit 17, performs modulation processing or the like on the obtained image signal, and generates a wireless signal modulated from the image signal. The wireless communication unit 16 transmits the wireless signal to the external transmitting/receiving unit 3 via the antenna 16a.

The control unit 17 controls each operation of the imaging units 11A and 11B and the wireless communication unit 16 that are components of the capsule endoscope 10, and controls input and output of signals between the components. Specifically, the control unit 17 causes the imaging element 15A to capture an image of the subject in the imaging view field illuminated by the illumination unit 13A and causes the imaging element 15B to capture an image of the subject in the imaging view field illuminated by the illumination unit 13B. The control unit 17 has a signal processing function to generate an image signal. The control unit 17 obtains in-vivo image data from the imaging elements 15A and 15B, and each time the control unit 17 obtains the in-vivo image data, the control unit 17 performs predetermined signal processing on the in-vivo image data and generates an image signal including the in-vivo image data. The control unit 17 controls the wireless communication unit 16 to sequentially wirelessly transmit the image signals to the outside in a chronological order.

The power source unit 18 is an electric accumulator such as a button-shaped battery or a capacitor, and the power source unit 18 is realized by including a switch unit such as a magnetic switch. The ON/OFF state of the power source unit 18 is switched by a magnetic field applied from the outside. During the ON-state, the power source unit 18 appropriately supplies the power of the electric accumulator to each component (imaging units 11A and 11B, wireless communication unit 16, and control unit 17) in the capsule endoscope 10. During the OFF-state, the power source unit 18 stops the supply of the power to each component in the capsule endoscope 10.

The permanent magnet 19 enables the magnetic guidance of the capsule endoscope 10 by the magnetic field generator 2. The permanent magnet 19 is disposed and fixed inside the capsule-shaped casing 12 in a state in which the permanent magnet 19 is relatively fixed to the above-described imaging units 11A and 11B. In this case, the permanent magnet 19 is magnetized in a known direction relatively fixed to the vertical direction of each imaging surface of the imaging elements 15A and 15B.

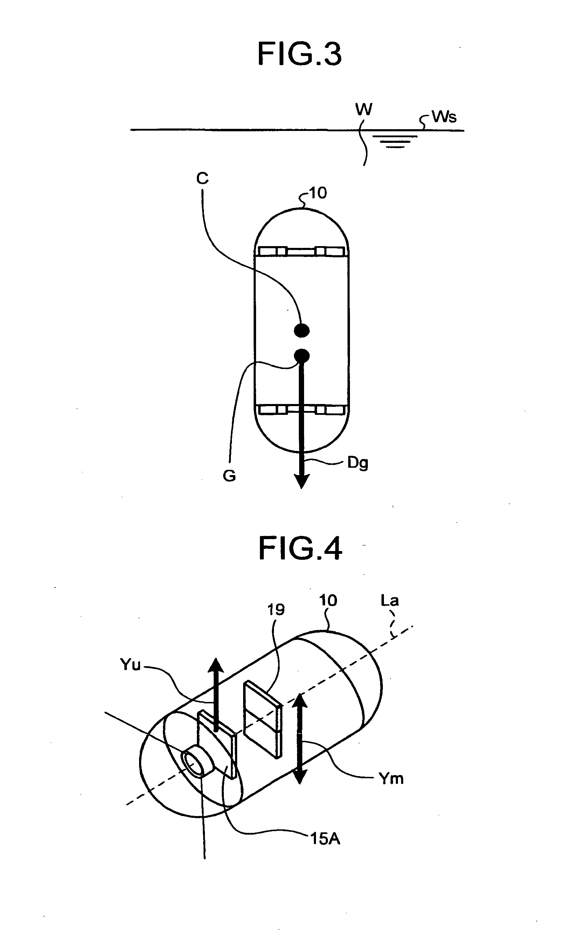

Here, a state of the capsule endoscope 10 in liquid W introduced into the subject will be described with reference to FIG. 3. FIG. 3 is a conceptual diagram explaining the state of the capsule endoscope 10 in the liquid W introduced into the subject. In an example illustrated in FIG. 3, a case in which the magnetic field for controlling the posture (orientation of the long axis La) of the capsule endoscope 10 is not applied to the permanent magnet 19 is illustrated.

The specific gravity relative to the liquid W of the capsule endoscope 10 illustrated in the first embodiment is designed to be approximately 1, so that the capsule endoscope 10 drifts in the liquid W as illustrated in FIG. 3. In this case, the center of gravity G of the capsule endoscope 10 is shifted from the geometrical center C of the capsule endoscope 10 along the long axis La (see FIG. 2) of the capsule endoscope 10. Specifically, the center of gravity G of the capsule endoscope 10 is set at a position that is on the long axis La and shifted from the geometrical center C of the capsule-shaped casing 12 toward the imaging unit 11B by adjusting the arrangement of the components of the capsule endoscope 10 such as the power source unit 18 and the permanent magnet 19. Thereby, the long axis La of the capsule endoscope 10 drifting in the liquid W becomes in parallel with the vertical direction (that is, the direction of the gravitational force). In other words, it is possible to drift the capsule endoscope 10 in the liquid W in an upright posture. The upright posture mentioned above is a posture in which the long axis La of the capsule-shaped casing 12 (a line connecting the geometrical center C and the center of gravity G) is substantially in parallel with the vertical direction. In the upright posture, the capsule endoscope 10 sets the imaging view field of the imaging unit 11A in the upper vertical direction and the imaging view field of the imaging unit 11B in the lower vertical direction. The long axis La of the capsule endoscope 10 is the central axis in the longitudinal direction of the capsule endoscope 10. The liquid W is a liquid harmless to the human body such as water or physiological saline.

As illustrated in FIG. 4, the permanent magnet 19 is fixed in the capsule-shaped casing 12 so that the magnetization direction Ym of the permanent magnet 19 has an angle relative to the long axis La of the capsule endoscope 10. For example, the permanent magnet 19 is fixed in the capsule-shaped casing 12 so that the magnetization direction Ym is perpendicular to the long axis La. Based on this configuration, when the capsule endoscope 10 drifts in the liquid W, the magnetization direction Ym of the permanent magnet 19 in the capsule endoscope 10 corresponds to a radial direction of the capsule endoscope 10. When the magnetic field for controlling the posture (orientation of the long axis La) of the capsule endoscope 10 is not applied to the permanent magnet 19, a plane that includes the magnetization direction Ym of the permanent magnet 19 and a direction (a displacement direction) from the geometrical center C of the capsule-shaped casing 12 to the center of gravity G of the capsule endoscope 10, which is shifted from the geometrical center C, is a vertical plane. Therefore, when applying a magnetic field, the posture of the capsule endoscope 10 changes so that the magnetization direction Ym is included in the vertical plane with respect to the magnetic field. The permanent magnet 19 moves following the magnetic field applied from the outside, and as a result, the magnetic guidance of the capsule endoscope 10 by the magnetic field generator 2 is implemented. In this case, the capsule endoscope 10 performs an operation to change at least one of the position, the posture, and the direction of the capsule endoscope 10 in the subject by an action of the permanent magnet 19.

Next, a relative relationship between the imaging elements 15A, 15B and the permanent magnet 19 that are contained in the capsule endoscope 10 will be described. As illustrated in FIGS. 2 and 4, the two imaging units 11A and 11B are disposed so that, for example, the optical central axes of the imaging elements 15A and 15B, which are respectively included in the imaging units 11A and 11B, overlap the long axis La and the image capturing directions of the imaging elements 15A and 15B are opposite to each other. In other words, the imaging units 11A and 11B are mounted so that the imaging surfaces of the imaging elements 15A and 15B are perpendicular to the long axis La. The permanent magnet 19 is disposed inside the capsule-shaped casing 12 in a state in which the permanent magnet 19 is relatively fixed to the imaging elements 15A and 15B. In this case, the permanent magnet 19 is disposed in the capsule endoscope 10 so that the magnetization direction Ym of the permanent magnet 19 is in parallel with the vertical direction Yu of the imaging surfaces of the imaging elements 15A and 15B as illustrated in FIG. 4. By setting the center of gravity G on the long axis La and mounting the imaging units 11A and 11B so that the imaging surfaces of the imaging elements 15A and 15B are perpendicular to the long axis La, it is possible to orthogonalize the imaging surfaces of the imaging elements 15A and 15B to a plane that includes the magnetization direction of the permanent magnet 19 and the displacement direction from the geometrical center C to the center of gravity G.

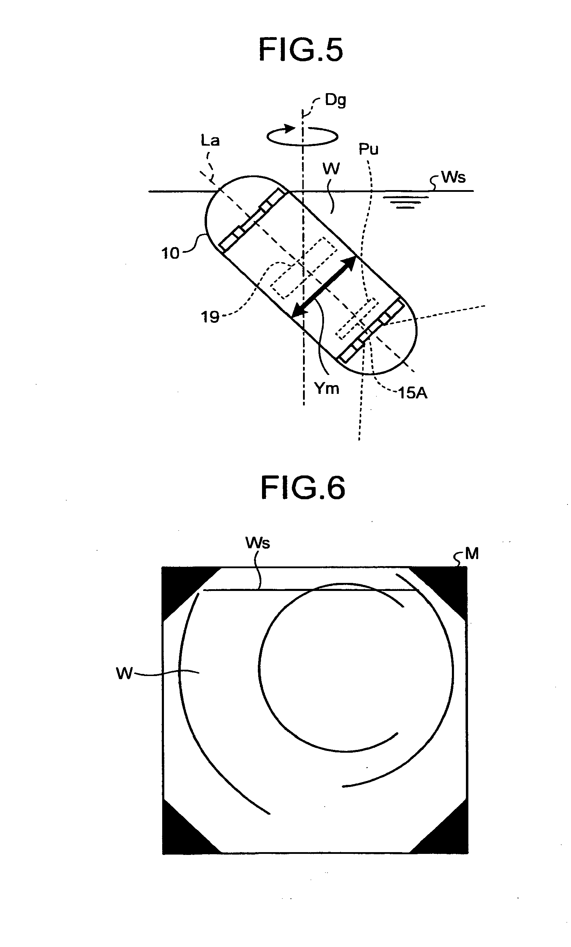

An angle of the long axis La of the capsule endoscope 10 relative to the direction of gravitational force Dg can be controlled by applying a magnetic field from the outside to the permanent magnet 19 of the capsule endoscope 10. As illustrated in FIG. 5, by applying a magnetic field whose magnetic line of force has an angle relative to the horizontal surface, the capsule endoscope 10 can be tilted from the direction of gravitational force Dg so that the magnetization direction Ym of the permanent magnet 19 is substantially in parallel with the magnetic line. Therefore, in a state in which the capsule endoscope 10 is tilted, only by applying a rotating magnetic field that rotates around the direction of gravitational force Dg and rotating the capsule endoscope 10 around the direction of gravitational force Dg as illustrated by an arrow, it is possible to easily obtain the in-vivo images around the capsule endoscope 10.

The display unit 5 displays the in-vivo image of the subject obtained by the capsule endoscope 10 in a display mode in which the up-down direction of the subject in the in-vivo image following the magnetic guidance of the capsule endoscope 10 is matched to the up-down direction of the display screen. As a result, as illustrated in FIG. 6, an image of the liquid surface Ws captured by an element in the upper portion Pu of the imaging element 15A of the capsule endoscope 10 is displayed in the upper portion of an image corresponding to the imaging unit 11A on the display screen M of the display unit 5. Since the magnetization direction Ym of the permanent magnet 19 is in parallel with the up-down direction Yu of the imaging surfaces of the imaging elements 15A and 15B, a direction in parallel with the magnetization direction Ym of the permanent magnet 19 matches the up-down direction of the display screen of the display unit 5.

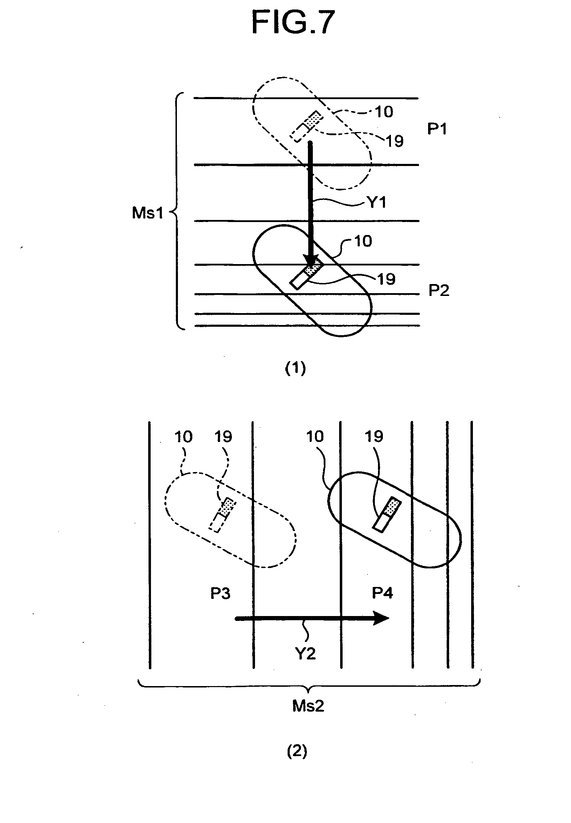

Next, the types of the magnetic field generated by the magnetic field generator 2 will be described. The magnetic field generator 2 can generate a gradient magnetic field in addition to a so-called uniform magnetic field. As the gradient magnetic field, there is a uniform gradient magnetic field having a substantially uniform magnetic field gradient. The uniform gradient magnetic field urges the permanent magnet 19 of the capsule endoscope 10 in a direction in which the distribution of magnetic field strength varies from sparse to dense. When the capsule endoscope 10 is desired to be urged downward in the vertical direction, as illustrated in FIG. 7(1), the magnetic field generator 2 generates a uniform gradient magnetic field Ms1 whose distribution of magnetic field strength varies from sparse to dense downward along the vertical axis. The permanent magnet 19 in the capsule endoscope 10 is urged by the uniform gradient magnetic field Ms1, and the capsule endoscope 10 at a position P1 moves to a vertically downward position P2 as illustrated by an arrow Y1. When the capsule endoscope 10 is desired to be urged rightward along the horizontal direction, as illustrated in FIG. 7(2), the magnetic field generator 2 generates a uniform gradient magnetic field Ms2 whose distribution of magnetic field strength varies from sparse to dense in a direction from left to right. The permanent magnet 19 in the capsule endoscope 10 is urged by the uniform gradient magnetic field Ms2, and the capsule endoscope 10 at a position P3 moves to a right position P4 as illustrated by an arrow Y2. The magnetic field generator 2 can also generate a peak magnetic field. The peak magnetic field is a magnetic field in which a magnetic field gradient is set so that a peak of magnetic field strength is located in a direction perpendicular to the horizontal surface, so that it is possible to attract the permanent magnet 19 to the peak position of the magnetic field strength and trap the capsule endoscope 10.

The capsule medical device guidance system 1 according to the first embodiment generates a magnetic field which makes a resultant force of the buoyancy of the capsule endoscope 10 and the gravity force and the vertical magnetic attracting force of the capsule endoscope 10 in the liquid substantially zero, in addition to the magnetic field for guiding the capsule endoscope 10 corresponding to an operation of a user from the operation input unit 7, and keeps the capsule endoscope 10 in a substantially static state in the liquid W.

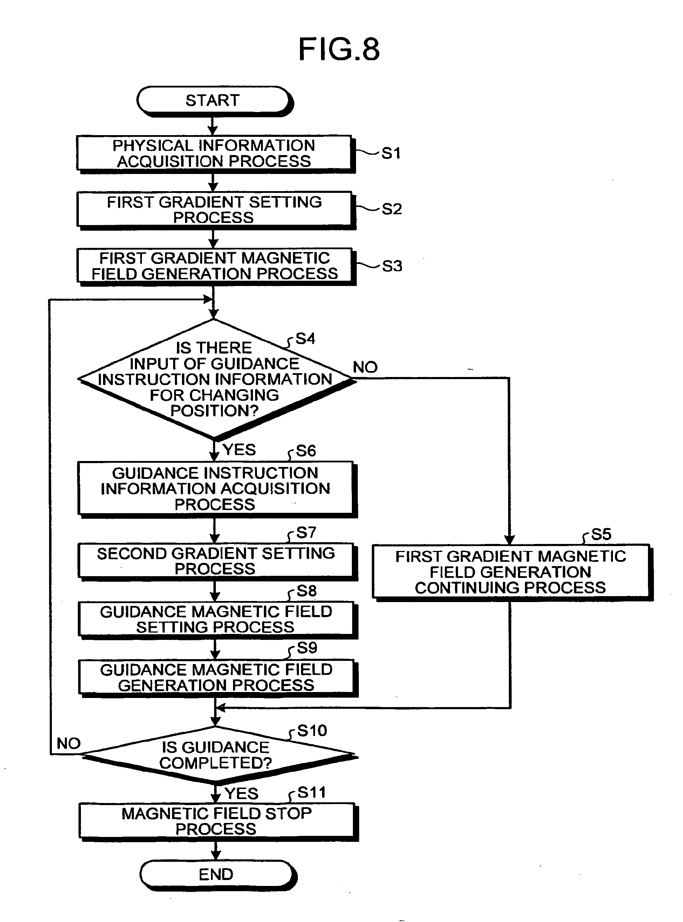

Now, a control process when the magnetic guidance is performed by the control unit 4 illustrated in FIG. 1 will be described with reference to FIG. 8. FIG. 8 is a flowchart illustrating a processing procedure when the magnetic guidance is performed by the control unit 4 illustrated in FIG. 1.

As illustrated in FIG. 8, first, in the magnetic field controller 45, the first magnetic field gradient setting unit 46 obtains a magnetic field for keeping the capsule endoscope 10 in a substantially static state in the liquid W, and controls the magnetic field generator 2 to generate the obtained magnetic field. The magnetic field for keeping the capsule endoscope 10 in a substantially static state in the liquid W is a uniform gradient magnetic field having a magnetic field gradient (the first magnetic field gradient) for generating a vertical magnetic attracting force which makes a resultant force of the buoyancy of the capsule endoscope 10 and the gravity force and the vertical magnetic attracting force of the capsule endoscope 10 in the liquid substantially zero. The gravity force of the capsule endoscope 10 and the buoyancy of the capsule endoscope 10 are determined by the mass and the volume of the capsule endoscope 10 and the density of the liquid W in which the capsule endoscope 10 floats. The magnetic attracting force of the capsule endoscope 10 is determined by the magnetic moment of the capsule endoscope 10. Therefore, the first magnetic field gradient setting unit 46 performs a physical information acquisition process to acquire physical information such as the mass, the volume, and the magnetic moment of the capsule endoscope 10 and the density of the liquid W in order to obtain the first magnetic field gradient (step S1). The physical information is inputted from the input unit 6 by an operation of a user using a mouse and a keyboard, and obtained in advance and stored in the storage unit 8. The physical information is read and acquired from the storage unit 8 by the magnetic field controller 45 when performing the physical information acquisition process.

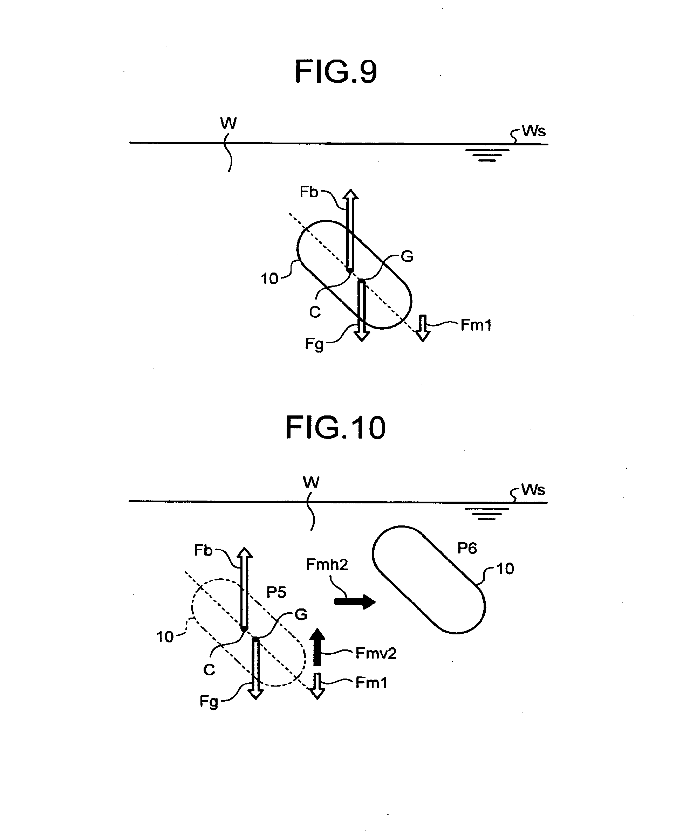

Next, the first magnetic field gradient setting unit 46 obtains a magnetic field gradient of a magnetic field that generates a vertical magnetic attracting force which makes a resultant force of the buoyancy of the capsule endoscope 10 and the gravity force and the vertical magnetic attracting force of the capsule endoscope 10 in the liquid W substantially zero on the basis of the acquired physical information such as the mass, the volume, and the magnetic moment of the capsule endoscope 10 and the density of the liquid W, and performs a first gradient setting process for setting the obtained magnetic field gradient as the first magnetic field gradient (step S2). Then, the magnetic field setting unit 48 sets a uniform gradient magnetic field having the first magnetic field gradient set by the first magnetic field gradient setting unit 46 as an initial condition of the magnetic field generated by the magnetic field generator 2, and the magnetic field controller 45 performs a first gradient magnetic field generation process for controlling the magnetic field generator 2 to apply a uniform gradient magnetic field having the first magnetic field gradient (step S3). As a result, as illustrated in FIG. 9, a vertical magnetic attracting force Fm1 which makes a resultant force of the buoyancy Fb of the capsule endoscope 10 and the gravity force Fg and the vertical magnetic attracting force of the capsule endoscope 10 in the liquid W substantially zero is applied to the capsule endoscope 10 along with the buoyancy Fb of the capsule endoscope 10 and the gravity force Fg of the capsule endoscope 10. Therefore, the capsule endoscope 10 is kept in a substantially static state in the liquid W by the magnetic attracting force Fm1.

Next, the magnetic field controller 45 determines whether or not there is an input of the guidance instruction information by the operation input unit 7 (step S4). If the magnetic field controller 45 determines that there is no input of the guidance instruction information by the operation input unit 7 (step S4: No), the magnetic field controller 45 performs a first gradient magnetic field generation continuing process for keeping generation of the uniform gradient magnetic field having the first magnetic field gradient by the magnetic field generator 2 without change (step S5).

On the other hand, if the magnetic field controller 45 determines that there is an input of the guidance instruction information for changing the position of the capsule endoscope 10 by the operation input unit 7 (step S4: Yes), the second magnetic field gradient setting unit 47 performs a guidance instruction information acquisition process for acquiring the guidance instruction information (step S6), obtains a magnetic field gradient of a magnetic field that generates a magnetic attracting force corresponding to the guidance instruction information, and performs a second gradient setting process for setting the magnetic field gradient as a second magnetic field gradient (step S7). Thereafter, the magnetic field setting unit 48 performs a guidance magnetic field setting process for setting a magnetic field having a magnetic field gradient obtained by adding the second magnetic field gradient set by the second magnetic field gradient setting unit 47 to the first magnetic field gradient set by the first magnetic field gradient setting unit 46 as a guidance magnetic field for the capsule endoscope 10 (step S8). Then the magnetic field controller 45 performs a guidance magnetic field generation process for controlling the magnetic field generator 2 to apply the guidance magnetic field set by the magnetic field setting unit 48 (step S9). As a result, as illustrated in FIG. 10, the guidance magnetic field corresponding to the guidance instruction information is applied to the capsule endoscope 10 which is kept in a substantially static state in which the vertical magnetic attracting force Fm1 which makes a resultant force of the buoyancy Fb of the capsule endoscope 10 and the gravity force Fg and the vertical magnetic attracting force of the capsule endoscope 10 in the liquid W substantially zero is applied to the capsule endoscope 10 along with the buoyancy Fb of the capsule endoscope 10 and the gravity force Fg of the capsule endoscope 10. Thereby, for example, a rightward magnetic attracting force Fmv2 and a vertically upward magnetic attracting force Fmh2 are applied to the capsule endoscope 10, and the capsule endoscope 10 is guided from a position P5 to a position P6 at upper light of the position P5 in accordance with the instruction of the guidance instruction information.

Then the magnetic field controller 45 determines whether or not the guidance process itself for the capsule endoscope 10 is completed on the basis of instruction information by the input unit 6 (step S10) following the first gradient magnetic field generation continuing process (step S5) or the guidance magnetic field generation process (step S9). If the magnetic field controller 45 determines that the guidance process is not completed (step S10: No), the magnetic field controller 45 returns to step S4 to continue the guidance process for the capsule endoscope 10, and determines whether or not there is an input of the guidance instruction information. If the magnetic field controller 45 determines that the guidance process is completed (step S10: Yes), the magnetic field controller 45 performs a magnetic field stop process for causing the magnetic field generator 2 to stop the magnetic field (step S11) and ends the magnetic guidance process of the capsule endoscope 10.

As illustrated in FIG. 8, in the first embodiment, the uniform gradient magnetic field having the first gradient capable of keeping the capsule endoscope 10 in a static state is automatically applied. In the first embodiment, when the guidance instruction information is inputted from the operation input unit 7, a magnetic field obtained by synthesizing the uniform gradient magnetic field having the first gradient and the magnetic field on which the guidance instruction information is reflected is applied, and when the guidance instruction information is not inputted from the operation input unit 7, the uniform gradient magnetic field having the first gradient is continuously applied without change.

In this way, in the first embodiment, even if a user does not operate directly, the uniform gradient magnetic field having the first gradient capable of keeping the capsule endoscope 10 in a static state is automatically applied as a base magnetic field, and if the guidance instruction information is inputted from the operation input unit 7 when the uniform gradient magnetic field having the first gradient is applied, the magnetic field on which the guidance instruction information is reflected is further applied. Therefore, according to the first embodiment, the magnetic attracting force of the magnetic field applied corresponding to the guidance instruction information of the operation input unit 7 is not consumed to counterbalance the countervailing force to the gravity force and the buoyancy of the capsule endoscope 10, and the magnetic attracting force is consumed substantially without change to guide the capsule endoscope 10. The force to guide the capsule endoscope 10 corresponds to approximately the amount of input. In other words, according to the first embodiment, an input to the operation input unit 7 is directly reflected on the guidance, so that it is possible to move the capsule endoscope 10 corresponding to the operation of the operation input unit 7, and the synthesis of the input in the horizontal direction and the input in the vertical direction substantially corresponds to the guidance direction. As a result, according to the first embodiment, it is possible to guide the capsule endoscope 10 in a direction in which the user operates, so that it is possible to provide a capsule medical device guidance system with high operability in which the operational feeling of the user matches the movement of the capsule endoscope 10.

Although not illustrated in FIG. 8, if the magnetic field controller 45 determines that there is an input of the guidance instruction information for changing the posture of the capsule endoscope 10 from the operation input unit 7, the magnetic field setting unit 48 changes the orientation of the magnetic field generated according to the inputted guidance instruction information.

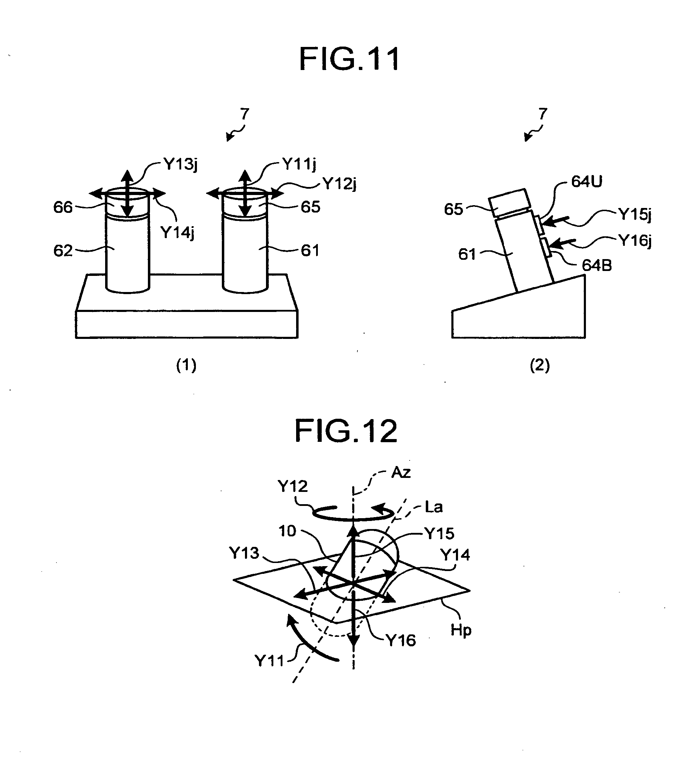

Here, the movement of the capsule endoscope 10 corresponding to the guidance operation of the operation input unit 7 illustrated in FIG. 1 will be described. FIG. 11(1) is a front view of the operation input unit 7; FIG. 11(2) is a right side view of the operation input unit 7; and FIG. 12 is a diagram illustrating the movements of the capsule endoscope 10 instructed by operations of each component of the operation input unit 7.

As illustrated in FIG. 11(1), the operation input unit 7 includes two joysticks 61 and 62 for three-dimensionally operating the magnetic guidance of the capsule endoscope 10 by the magnetic field generator 2. The joysticks 61 and 62 are capable of a tilt operation in the vertical direction and the horizontal direction.

As illustrated in FIG. 11(2), an up button 64U and a down button 64B are provided on the rear surface of the joystick 61. When the up button 64U is pressed, the up button 64U inputs guidance instruction information for instructing an upward guidance of the capsule endoscope 10 into the control unit 4, and when the down button 64B is pressed, the down button 64B inputs guidance instruction information for instructing a downward guidance of the capsule endoscope 10 into the control unit 4. A capture button 65 is provided on the top of the joystick 61. When the capture button 65 is pressed, the capture button 65 captures an in-vivo image displayed on the display unit 5. An approach button 66 is provided on the top of the joystick 62. When the approach button 66 is pressed, the approach button 66 inputs guidance instruction information for guiding the capsule endoscope 10 so that the imaging unit 11A of the capsule endoscope 10 approaches an imaging object of the imaging unit 11A into the control unit 4.

As illustrated in FIG. 11(1), the tilt direction in the vertical direction of the joystick 61 illustrated by the arrow Y11j corresponds to a tilting guidance direction in which the distal end of the capsule endoscope 10 oscillates through the vertical axis Az as illustrated by the arrow Y11 in FIG. 12. When the guidance instruction information corresponding to the tilt operation of the joystick 61 illustrated by the arrow Y11j is inputted into the control unit 4 from the operation input unit 7, on the basis of the guidance instruction information, the magnetic field setting unit 48 calculates a guidance direction of the distal end of the capsule endoscope 10 on the absolute coordinate system in accordance with the tilt direction of the joystick 61 and calculates a guidance speed in accordance with the tilt operation of the joystick 61. Then, for example, the magnetic field setting unit 48 changes the orientation of the magnetic field to the calculated guidance direction in accordance with the calculated guidance speed.

As illustrated in FIG. 11(1), the tilt direction in the horizontal direction of the joystick 61 illustrated by the arrow Y12j corresponds to a rotation guidance direction in which the capsule endoscope 10 rotates around the vertical axis Az as illustrated by the arrow Y12 in FIG. 12. When the guidance instruction information corresponding to the tilt operation of the joystick 61 illustrated by the arrow Y12j is inputted into the control unit 4 from the operation input unit 7, on the basis of the guidance instruction information, the magnetic field setting unit 48 calculates a guidance direction of the distal end of the capsule endoscope 10 on the absolute coordinate system in accordance with the tilt direction of the joystick 61, calculates a guidance speed in accordance with the tilt operation of the joystick 61, and, for example, changes the orientation of the magnetic field to the calculated guidance direction in accordance with the calculated guidance speed.

As illustrated in FIG. 11(1), the tilt direction in the vertical direction of the joystick 62 illustrated by the arrow Y13j corresponds to a horizontal backward guidance direction or a horizontal forward guidance direction in which the capsule endoscope 10 moves in a direction indicated by a line obtained by projecting the long axis La of the capsule endoscope 10 to the horizontal surface Hp as illustrated by the arrow Y13 in FIG. 12. When the guidance instruction information corresponding to the tilt operation of the joystick 62 illustrated by the arrow Y13j is inputted into the control unit 4 from the operation input unit 7, on the basis of the guidance instruction information, the second magnetic field gradient setting unit 47 calculates a guidance direction and a guidance position of the distal end of the capsule endoscope 10 on the absolute coordinate system in accordance with the tilt direction of the joystick 62, calculates a generating force in accordance with the tilt operation of the joystick 62, causes the magnetic field generator 2 to generate, for example, a uniform gradient magnetic field having a gradient corresponding to the calculated guidance direction, and sets the distribution of the magnetic field strength so that urging can be performed by the calculated generating force.

As illustrated in FIG. 11(1), the tilt direction in the horizontal direction of the joystick 62 illustrated by the arrow Y14j corresponds to a horizontal right guidance direction or a horizontal left guidance direction in which the capsule endoscope 10 moves on the horizontal surface Hp in a direction perpendicular to a direction indicated by a line obtained by projecting the long axis La to the horizontal surface Hp as illustrated by the arrow Y14 in FIG. 12. When the guidance instruction information corresponding to the tilt operation of the joystick 62 illustrated by the arrow Y14j is inputted into the control unit 4 from the operation input unit 7, on the basis of the guidance instruction information, the second magnetic field gradient setting unit 47 calculates a guidance direction and a guidance position of the distal end of the capsule endoscope 10 on the absolute coordinate system in accordance with the tilt direction of the joystick 62, calculates a generating force in accordance with the tilt operation of the joystick 62, causes the magnetic field generator 2 to generate, for example, a uniform gradient magnetic field having a gradient corresponding to the calculated guidance direction, and sets the distribution of the magnetic field strength so that urging can be performed by the calculated generating force.

An up button 64U and a down button 64B are provided on the rear surface of the joystick 62. When the up button 64U is pressed as illustrated by the arrow Y15j in FIG. 11(2), an up operation is instructed in which the capsule endoscope 10 moves upward along the vertical axis Az as illustrated by the arrow Y15 in FIG. 12. When the down button 64B is pressed as illustrated by the arrow Y16j in FIG. 11(2), a down operation is instructed in which the capsule endoscope 10 moves downward along the vertical axis Az as illustrated by the arrow Y16 in FIG. 12. When the guidance instruction information corresponding to the pressing operation of the up button 64U or the down button 64B illustrated by the arrows Y15j and Y16j is inputted into the control unit 4 from the operation input unit 7, on the basis of the guidance instruction information, the second magnetic field gradient setting unit 47 calculates a guidance direction of the distal end of the capsule endoscope 10 on the absolute coordinate system according to which button is pressed, and, for example, causes the magnetic field generator 2 to generate a uniform gradient magnetic field having a gradient along the vertical axis Az according to the calculated guidance direction. When the up button 64U is pressed, the magnetic field generator 2 generates a uniform gradient magnetic field having a gradient in which the density of the magnetic field increases in the upward direction of the vertical axis Az, and thereby moves the capsule endoscope 10 as illustrated by the arrow Y15. When the down button 64B is pressed, the magnetic field generator 2 generates a uniform gradient magnetic field having a gradient in which the density of the magnetic field increases in the downward direction of the vertical axis Az, and thereby moves the capsule endoscope 10 as illustrated by the arrow Y16.

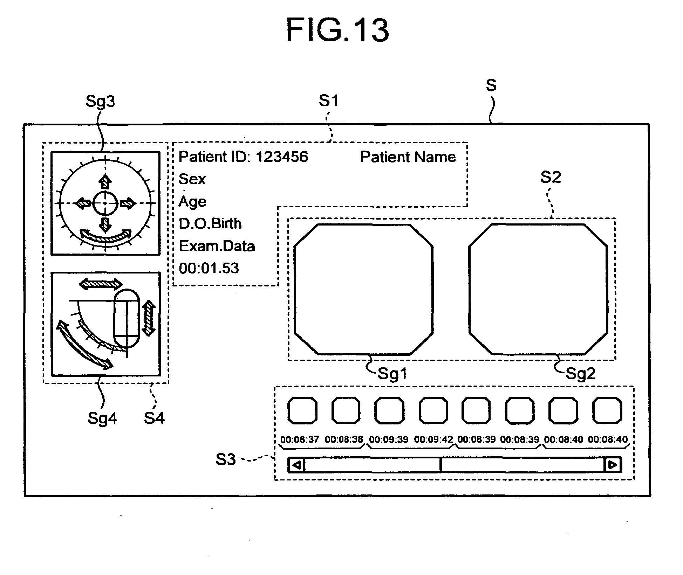

On the display unit 5, for example, a menu screen S illustrated in FIG. 13 is displayed. In the menu screen S, subject information such as patient name, patient ID, date of birth, sex, and age of the subject is displayed in the upper left area S1, a biological image Sg1 captured by the imaging unit 11A is displayed in the left of the center area S2, a biological image Sg2 captured by the imaging unit 11B is displayed in the right of the center area S2, reduced images of the images captured by the pressing operation of the capture button 65 are displayed in the area S3 below the area S2 along with the times when the images are captured, and a posture diagram Sg3 in a vertical plane and a posture diagram Sg4 in a horizontal plane are displayed as posture diagrams of the capsule endoscope 10 in the left area S4. The postures of the capsule endoscope 10 displayed in the posture diagrams Sg3 and Sg4 display postures corresponding to the guidance instruction information of the operation input unit 7. In the first embodiment, as described above, the amount of input from the operation input unit 7 is directly reflected on the force of guiding, so that the displayed postures of the capsule endoscope 10 are assumed to be substantially the same as the actual posture of the capsule endoscope 10. Therefore, support of the guidance instruction of the user is improved. In the posture diagrams Sg3 and Sg4, directions in which the capsule endoscope 10 can be guided are indicated by arrows, and when an operation of any guidance direction is inputted, the display color of the arrow corresponding to the inputted guidance direction is changed to support the operation of the user.

In the first embodiment, each operation of the operation input unit 7 may be set in association with the guidance operations of the capsule endoscope 10 so that the capsule endoscope 10 can be guided along the plane perpendicular to the long axis of the capsule endoscope 10 instead of the horizontal surface Hp. Hereinafter, the movements of the capsule endoscope 10 corresponding to the guidance operations in which the capsule endoscope 10 is guided along the plane perpendicular to the long axis of the capsule endoscope 10 will be described.

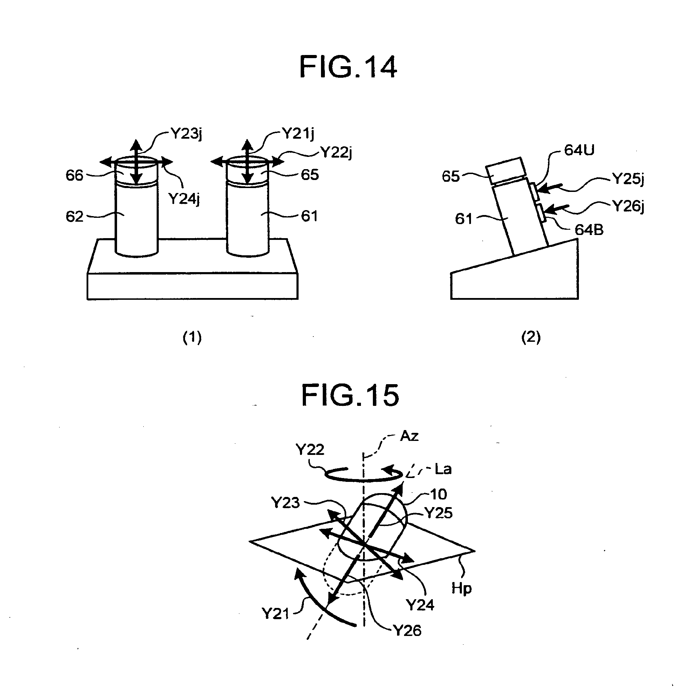

FIG. 14(1) is a front view of the operation input unit 7; FIG. 14(2) is a right side view of the operation input unit; and FIG. 15 is a diagram illustrating the contents of operation of the capsule endoscope 10 under instructions made with each constituent section of the operation input unit 7.

As illustrated in FIG. 14(1), as the tilt direction in the vertical direction of the joystick 62 illustrated by the arrow Y23j indicates a down guidance direction or an up guidance direction in which the capsule endoscope 10 moves in a direction indicated by the arrow Y23 in a plane perpendicular to the long axis La of the capsule endoscope 10 illustrated in FIG. 15. When the operation information corresponding to the tilt operation of the joystick 62 illustrated by the arrow Y23j is inputted into the control unit 4 from the operation input unit 7, on the basis of the operation information, the magnetic field setting unit 48 calculates a guidance direction of the distal end of the capsule endoscope 10 on the absolute coordinate system in accordance with the tilt direction of the joystick 62, calculates a guidance speed in accordance with the tilt operation of the joystick 62, and, for example, changes the orientation of the magnetic field to the calculated guidance direction in accordance with the calculated guidance speed.

As illustrated in FIG. 14(1), the tilt direction in the horizontal direction of the joystick 62 illustrated by the arrow Y24j indicates a right guidance direction or a left guidance direction in which the capsule endoscope 10 moves in a direction indicated by the arrow Y24 in a plane perpendicular to the long axis La of the capsule endoscope 10 illustrated in FIG. 15. When the operation information corresponding to the tilt operation of the joystick 62 illustrated by the arrow Y24j is inputted into the control unit 4 from the operation input unit 7, on the basis of the operation information, the second magnetic field gradient setting unit 47 calculates a guidance direction of the distal end of the capsule endoscope 10 on the absolute coordinate system in accordance with the tilt direction of the joystick 62, calculates a generating force in accordance with the tilt operation of the joystick 61, and causes the magnetic field generator 2 to generate a uniform gradient magnetic field having a gradient whose direction corresponds to the calculated guidance direction and which corresponds to the calculated generating force.

Further, as illustrated in FIG. 14(2), when the up button 64U or the down button 64B is pressed as illustrated by the arrows Y25j and Y26j, a forward guidance direction or a backward guidance direction is instructed in which the imaging elements 15A and 15B move forward or backward as illustrated by the arrows Y25 and Y26 along the long axis La of the capsule endoscope 10 illustrated in FIG. 15. When the operation information corresponding to the pressing operation of the up button 64U or the down button 64B illustrated by the arrows Y25j and Y26j is inputted into the control unit 4 from the operation input unit 7, on the basis of the operation information, the second magnetic field gradient setting unit 47 calculates an guidance direction of the distal end of the capsule endoscope 10 on the absolute coordinate system according to which button is pressed, and causes the magnetic field generator 2 to generate a uniform gradient magnetic field having a gradient along the long axis La according to the calculated guidance direction.

As illustrated in FIG. 14(1), the tilt direction in the vertical direction of the joystick 61 illustrated by the arrow Y21j corresponds to a tilting guidance direction in which the distal end of the capsule endoscope 10 oscillates through the vertical axis Az as illustrated by the arrow Y21 in FIG. 15, and the tilt direction in the horizontal direction of the joystick 61 illustrated by the arrow Y22j corresponds to a rotation guidance direction in which the capsule endoscope 10 rotates around the vertical axis Az as illustrated by the arrow Y22 in FIG. 15.

Here, if it is set so that the capsule endoscope is guided along the plane perpendicular to the long axis of the capsule endoscope, in a conventional operation, a part of the magnetic attracting force in the vertical direction of the generated magnetic field is consumed as a countervailing force to the gravity force and the buoyancy of the capsule endoscope and the guidance in the vertical direction is cancelled, so that, in particular, when the capsule endoscope is guided in the direction of the long axis La as illustrated by the arrows Y25 and Y26 in FIG. 15, the capsule endoscope cannot be guided in the direction of the long axis La as instructed by the operation. On the other hand, in the first embodiment, even if a user does not operate, while the uniform gradient magnetic field having the first gradient capable of keeping the capsule endoscope 10 in a static state is automatically applied as a base magnetic field, the magnetic field on which the guidance instruction information for changing the position of the capsule endoscope 10 from the operation input unit 7 is reflected is further applied. Therefore, according to the first embodiment, the magnetic attracting force of the magnetic field applied corresponding to the guidance instruction information for changing the position of the capsule endoscope 10 from the operation input unit 7 is not consumed as the countervailing force to the gravity force and the buoyancy of the capsule endoscope 10, and the magnetic attracting force is consumed substantially without change to guide the capsule endoscope 10. As a result, according to the first embodiment, it is possible to guide the capsule endoscope 10 in a direction in which the user operates, so that it is possible to provide a capsule medical device guidance system with high operability in which the operational feeling of the user matches the movement of the capsule endoscope 10.

In the first embodiment, in the physical information acquisition process (step S1) illustrated in FIG. 8, the capsule endoscope 10 may be kept in a static state at a higher degree of accuracy by measuring the volume and the mass for each capsule endoscope 10, inputting the measurement result, and setting the first magnetic field gradient for each capsule endoscope 10. Also, in the physical information acquisition process (step S1) illustrated in FIG. 8, the volume and the mass of the capsule endoscope 10 may be measured for each production lot instead of for each capsule endoscope 10, and the measurement result may be inputted to set the first magnetic field gradient for each capsule endoscope 10. Also, in the physical information acquisition process (step S1) illustrated in FIG. 8, physical information such as the mass, the volume, and the magnetic moment of the capsule endoscope 10 and the density of the liquid W for a large intestine or a stomach is obtained in advance and the physical information is stored in the storage unit 8 in association with each intended purpose, and the magnetic field controller 45 may read the physical information for a large intestine from the storage unit 8 when the physical information for a large intestine is selected by an operation of the input unit 6 by a user, and may read the physical information for a stomach from the storage unit 8 when the physical information for a stomach is selected by an operation of the input unit 6 by a user.

### Second Embodiment

### (Second Embodiment)

Next, a second embodiment will be described. In the second embodiment, a case will be described in which a function to detect the movement of the capsule endoscope in the vertical direction is added and the first magnetic field gradient is fine-tuned so that the capsule endoscope is reliably kept in a static state.

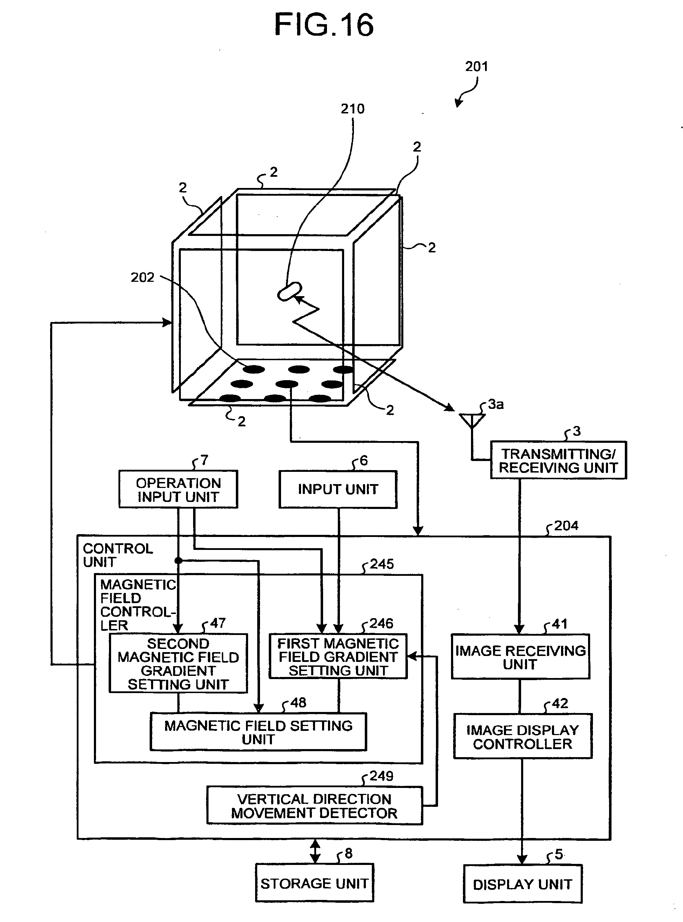

FIG. 16 is a schematic diagram illustrating an entire configuration of a capsule medical device guidance system according to the second embodiment. As illustrated in FIG. 16, a capsule medical device guidance system 201 according to the second embodiment has a configuration which uses a capsule endoscope 210 instead of the capsule endoscope 10 illustrated in FIG. 1 and a control unit 204 instead of the control unit 4 illustrated in FIG. 1 and further includes a plurality of magnetic field detectors 202. Compared with the control unit 4 illustrated in FIG. 1, the control unit 204 further includes a vertical direction movement detector 249 and includes a magnetic field controller 245 instead of the magnetic field controller 45 illustrated in FIG. 1. The magnetic field controller 245 includes a first magnetic field gradient setting unit 246 instead of the first magnetic field gradient setting unit 46.

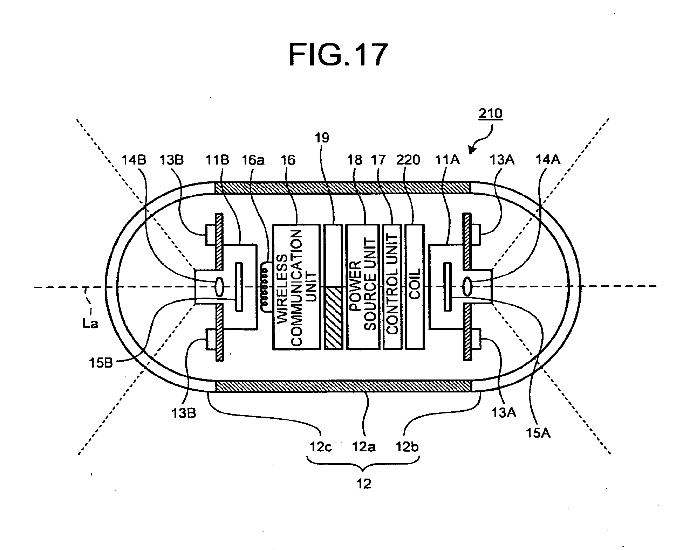

As illustrated in FIG. 17, the capsule endoscope 210 further includes a coil 220 that generates an alternating current magnetic field compared with the capsule endoscope 10 illustrated in FIG. 2. The magnetic field detector 202 illustrated in FIG. 16 includes a plurality of magnetic field detection coils. The magnetic field detector 202 detects the alternating current magnetic field generated by the coil 220 of the capsule endoscope 210 and outputs the detection result to the control unit 204.

The vertical direction movement detector 249 detects the vertical movement of the capsule endoscope 210 on the basis of the detection results outputted from the magnetic field detectors 202 and detects whether or not the capsule endoscope 210 moves in the vertical direction. For example, the vertical direction movement detector 249 continuously calculates the position coordinates and the orientation vector of the capsule endoscope 210 in three-dimensional space on the basis of the continuous detection results of the magnetic field detectors 202.

The first magnetic field gradient setting unit 246 obtains the first magnetic field gradient which is a magnetic field gradient capable of keeping the capsule endoscope 210 in a static state by using the detection result of the vertical direction movement detector 249 along with the physical parameter of the capsule endoscope 210 and the physical parameter of the liquid. The first magnetic field gradient setting unit 246 fine-tunes the first magnetic field gradient obtained based on the physical parameter of the capsule endoscope 210 and the physical parameter of the liquid on the basis of the detection result of the vertical direction movement detector 249, so that the capsule endoscope 210 can be kept in a static state at a higher degree of accuracy.

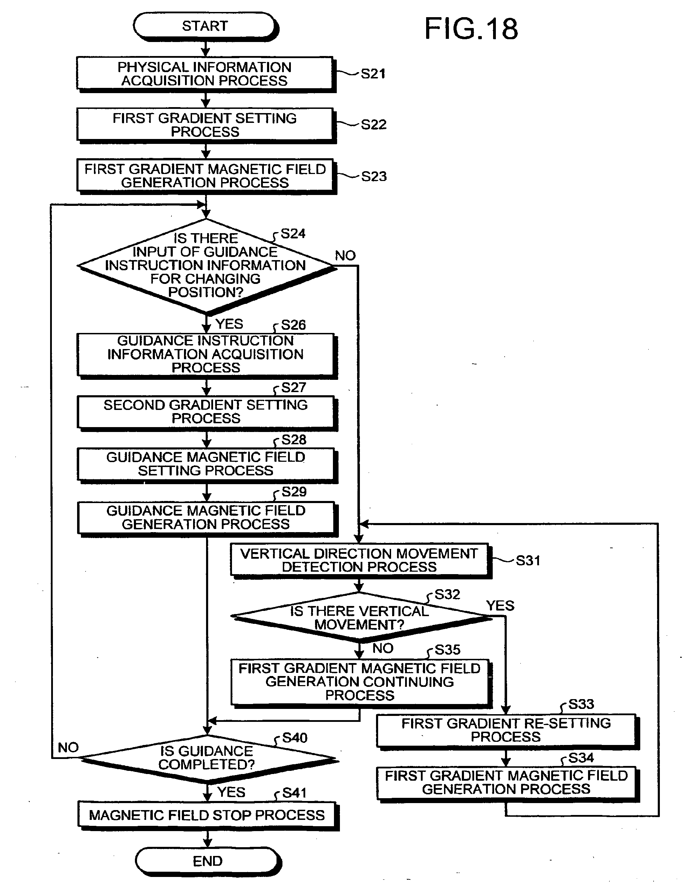

Next, a control process when the magnetic guidance is performed by the control unit 204 illustrated in FIG. 16 will be described. FIG. 18 is a flowchart illustrating a processing procedure when the magnetic guidance is performed by the control unit 204 illustrated in FIG. 16.

As illustrated in FIG. 18, the first magnetic field gradient setting unit 246 performs a physical information acquisition process (step S21), a first gradient setting process (step S22), and a first gradient magnetic field generation process (step S23) in the same processing procedure as that of steps S21 to S23 illustrated in FIG. 8 in order to obtain the first magnetic field gradient. Next, the magnetic field controller 245 determines whether or not there is an input of the guidance instruction information for changing the position of the capsule endoscope 210 by the operation input unit 7 (step S24). In the physical information acquisition process, all physical information not necessarily has to be acquired, and at least the magnetic moment of the capsule endoscope 210 only has to be acquired.

If the magnetic field controller 245 determines that there is no input of the guidance instruction information for changing the position of the capsule endoscope 210 by the operation input unit 7 (step S24: No), the vertical direction movement detector 249 performs a vertical direction movement detection process for detecting the vertical movement of the capsule endoscope 210 (step S31) and outputs the detection result to the first magnetic field gradient setting unit 246 in order to fine-tune the first magnetic field gradient so that the capsule endoscope 210 can be kept in a static state at a higher degree of accuracy. The first magnetic field gradient setting unit 246 determines whether or not there is a vertical movement of the capsule endoscope 210 on the basis of the detection result of the vertical direction movement detector 249 (step S32). In this case, the first magnetic field gradient setting unit 246 determines that there is no vertical movement when the vertical position of the capsule endoscope 210 does not change at all, and further the first magnetic field gradient setting unit 246 may determine that there is no vertical movement when the amount of vertical position change of the capsule endoscope 210 is within a predetermined range.

If the first magnetic field gradient setting unit 246 determines that there is a vertical movement of the capsule endoscope 210 (step S32: Yes), the first magnetic field gradient setting unit 246 performs a first gradient re-setting process for re-setting the first magnetic field gradient (step S33). In the first gradient re-setting process, the first magnetic field gradient setting unit 246 re-sets the first magnetic field gradient so that the capsule endoscope 210 moves in the opposite direction to the vertical guidance direction of the capsule endoscope 210.

For example, when the first magnetic field gradient is set so that the distribution of the magnetic field strength varies from sparse to dense downward along the vertical axis and the capsule endoscope 210 moves downward along the vertical axis, the vertical axis downward magnetic attracting force is too large, so that the first magnetic field gradient setting unit 246 reduces the first magnetic field gradient by one level to soften the first magnetic field gradient. On the other hand, when the first magnetic field gradient is set so that the distribution of the magnetic field strength varies from sparse to dense downward along the vertical axis and the capsule endoscope 210 moves upward along the vertical axis, the vertical axis downward magnetic attracting force is insufficient, so that the first magnetic field gradient setting unit 246 increases the first magnetic field gradient by one level to sharpen the first magnetic field gradient. When the first magnetic field gradient is set so that the distribution of the magnetic field strength varies from sparse to dense upward along the vertical axis and the capsule endoscope 210 moves upward along the vertical axis, the vertical axis upward magnetic attracting force is too large, so that the first magnetic field gradient setting unit 246 reduces the first magnetic field gradient by one level to soften the first magnetic field gradient. On the other hand, when the first magnetic field gradient is set so that the distribution of the magnetic field strength varies from sparse to dense upward along the vertical axis and the capsule endoscope 210 moves downward along the vertical axis, the vertical axis upward magnetic attracting force is insufficient, so that the first magnetic field gradient setting unit 246 increases the first magnetic field gradient by one level to sharpen the first magnetic field gradient.

Thereafter, the magnetic field controller 245 performs the first gradient magnetic field generation process for controlling the magnetic field generator 2 to apply a uniform gradient magnetic field having the first magnetic field gradient re-set by the first magnetic field gradient setting unit 246 (step S34). Then the control unit 204 returns to step S31 and repeats steps S31 to S34 until the vertical movement of the capsule endoscope 210 disappears.

If the first magnetic field gradient setting unit 246 determines that there is no vertical movement of the capsule endoscope 210 (step S32: No), this is a case in which the first magnetic field gradient is set to a gradient capable of keeping the capsule endoscope 210 in a static state, so that it is not necessary to fine-tune the first magnetic field gradient. Thereby, the first gradient magnetic field generation continuing process is performed in which the magnetic field generator 2 keeps generation of the uniform gradient magnetic field having the first magnetic field gradient without change (step S35).

On the other hand, if the magnetic field controller 245 determines that there is an input of the guidance instruction information for changing the position of the capsule endoscope 210 by the operation input unit 7 (step S24: Yes), in the same processing procedure as that of steps S6 to S9 illustrated in FIG. 8, the guidance instruction information acquisition process (step S26) and the second gradient setting process (step S27) are performed by the second magnetic field gradient setting unit 47, the guidance magnetic field setting process (step S28) is performed by the magnetic field setting unit 48, and the guidance magnetic field generation process (step S29) is performed by the magnetic field controller 245. Next, in the same manner as in steps S10 and S11 illustrated in FIG. 8, the guidance completion determination process (step S40) and the magnetic field stop process (step S41) are performed by the magnetic field controller 245.

Although not illustrated in FIG. 18, if the magnetic field controller 245 determines that there is an input of the guidance instruction information for changing the posture of the capsule endoscope 210 from the operation input unit 7, the magnetic field setting unit 48 changes the orientation of the magnetic field generated according to the inputted guidance instruction information.

As illustrated in FIG. 18, in the second embodiment, if the guidance instruction information for changing the position of the capsule endoscope 210 is not inputted from the operation input unit 7, the first magnetic field gradient is automatically fine-tuned until the vertical movement of the capsule endoscope 210 stops, so that it is possible to keep the capsule endoscope in a static state at a higher degree of accuracy when the magnetic guidance is not performed. Therefore, reflectiveness of an input into the operation input unit 7 to the magnetic guidance can be enhanced.

Also, in the second embodiment, the first magnetic field gradient is automatically fine-tuned until the vertical movement of the capsule endoscope 210 stops, so that it is possible to set the first magnetic field gradient at a high degree of accuracy even when all the physical information cannot be acquired.

The magnetic field generator 2 cannot generate a gradient magnetic field having a completely uniform magnetic field gradient in a space in which the capsule endoscope 210 is guided, and the magnetic field gradient varies depending on the position in the space. As a result, the magnetic attracting force generated to the capsule endoscope 210 is subtly changed depending on the position of the capsule endoscope 210, and the capsule endoscope 210 in the liquid W is affected by the subtle change of the magnetic attracting force. In the second embodiment, the first magnetic field gradient is automatically fine-tuned until the vertical movement of the capsule endoscope 210 stops, so that it is possible to keep the capsule endoscope in a static state at a high degree of accuracy regardless of the position of the capsule endoscope 210.

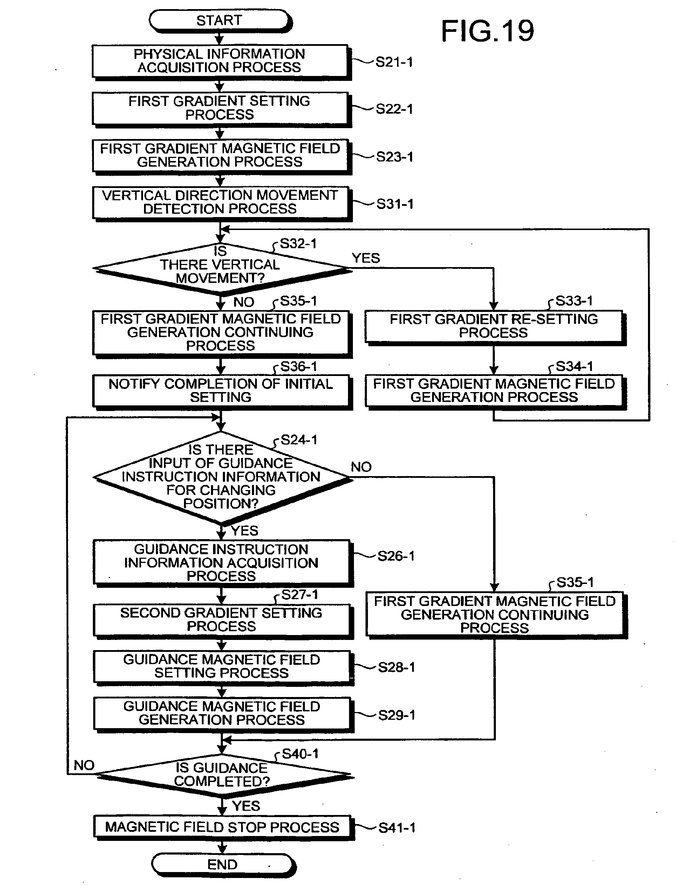

In the second embodiment, as an initial setting, the first magnetic field gradient may be fine-tuned until the vertical movement of the capsule endoscope 210 stops, and then the operation by the operation input unit 7 may be released. In this case, as illustrated in FIG. 19, to obtain the first magnetic field gradient, in the same processing procedure as that of steps S1 to S3 illustrated in FIG. 8, the physical information acquisition process (step S21-1), the first gradient setting process (step S22-1), and the first gradient magnetic field generation process (step S23-1) are performed, and then, in the same processing procedure as that of steps S31 to S35 illustrated in FIG. 18, the vertical direction movement detection process (step S31-1), the determination process of the vertical movement (step S32-1), the first gradient re-setting process (step S33-1), the first gradient magnetic field generation process (step S34-1), and the first gradient magnetic field generation continuing process (step S35-1) are performed. The control unit 204 repeats steps S31-1 to S34-1 until the vertical movement of the capsule endoscope 210 disappears.

After the first gradient magnetic field generation continuing process (step S35-1), in other words, after the vertical movement of the capsule endoscope 210 stops, the control unit 204 notifies the user of the completion of the initial setting (step S36-1). In this case, the control unit 204 may cause the display unit 5 to display a notice indicating that the initial setting is completed and the guidance instruction by the operation input unit 7 can be started, or cause a speaker not illustrated in the drawings to make an audio output indicating that the initial setting is completed and the guidance instruction by the operation input unit 7 can be started.

As a result, the user starts an operation using the operation input unit 7, and an input of the guidance instruction information from the operation input unit 7 to the control unit 204 is started by the operation of the operation input unit 7 by the user. Therefore, in the control unit 204, in the same processing procedure as that of steps S4 to S11 illustrated in FIG. 8, the guidance instruction information input determination process (step S24-1), the first gradient magnetic field generation continuing process (step S35-1), the guidance instruction information acquisition process (step S26-1), the second gradient setting process (step S27-1), the guidance magnetic field setting process (step S28-1), the guidance magnetic field generation process (step S29-1), the guidance completion determination process (step S40-1), and the magnetic field stop process (step S41-1) are performed.

Although not illustrated in FIG. 19, if the magnetic field controller 245 determines that there is an input of the guidance instruction information for changing the posture of the capsule endoscope 210 from the operation input unit 7, the magnetic field setting unit 48 changes the orientation of the magnetic field generated according to the inputted guidance instruction information.

### First Modified Example of the Second Embodiment

### (Modification 1 of Second Embodiment)

Next, a first modified example of the second embodiment will be described. In the first modified example of the second embodiment, a case will be described in which, even when the generated magnetic field varies depending on the position in the space, the position of the capsule endoscope is detected and the first magnetic field gradient is fine-tuned so that the capsule endoscope can be correctly kept in a static state on the basis of the detected position of the capsule endoscope and magnetic field distribution information.

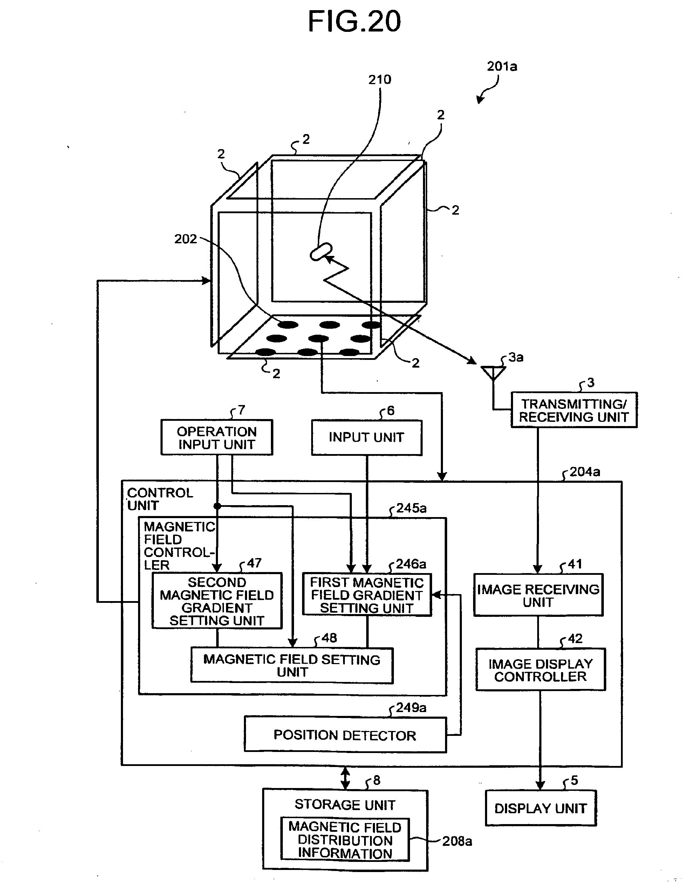

FIG. 20 is a schematic diagram illustrating an entire configuration of a capsule medical device guidance system according to the first modified example of the second embodiment. As illustrated in FIG. 20, a capsule medical device guidance system 201a according to the first modified example of the second embodiment has a configuration including a control unit 204a instead of the control unit 204 illustrated in FIG. 16 compared with the second embodiment. The storage unit 8 stores magnetic field distribution information 208a obtained in advance. The magnetic field generator 2 cannot generate a gradient magnetic field having a completely uniform magnetic field gradient in a space in which the capsule endoscope 210 is guided, and the magnetic field gradient varies depending on the position in the space. The storage unit 8 stores a distribution state of the magnetic field gradients at positions in the space in which the capsule endoscope 210 is guided as the magnetic field distribution information 208a.

Compared with the control unit 204 illustrated in FIG. 16, the control unit 204a includes a position detector 249a instead of the vertical direction movement detector 249 and a magnetic field controller 254a instead of the magnetic field controller 245. The magnetic field controller 245a includes a first magnetic field gradient setting unit 246a instead of the first magnetic field gradient setting unit 246.

The position detector 249a continuously calculates the position coordinates and the orientation vector of the capsule endoscope 210 in three-dimensional space on the basis of the continuous detection results of the magnetic field detectors 202. The first magnetic field gradient setting unit 246a obtains and sets the first magnetic field gradient which is a magnetic field gradient capable of keeping the capsule endoscope 210 in a static state by using the position detection result of the position detector 249a and the magnetic field distribution information 208a stored in the storage unit 8 along with the physical parameter of the capsule endoscope 210 and the physical parameter of the liquid. The first magnetic field gradient setting unit 246a fine-tunes the first magnetic field gradient obtained based on the physical parameter of the capsule endoscope 210 and the physical parameter of the liquid on the basis of the position detection result of the position detector 249a and the magnetic field distribution information 208a stored in the storage unit 8, so that the capsule endoscope 210 can be kept in a static state at a higher degree of accuracy. Specifically, the first magnetic field gradient setting unit 246a refers to the magnetic field distribution information 208a, obtains a change value indicating how much the magnetic field gradient of the magnetic field generated by the magnetic field generator 2 changes at the position detected by the position detector 249a, and changes the first magnetic field gradient obtained based on the physical parameter of the capsule endoscope 210 and the physical parameter of the liquid according to the obtained change value.

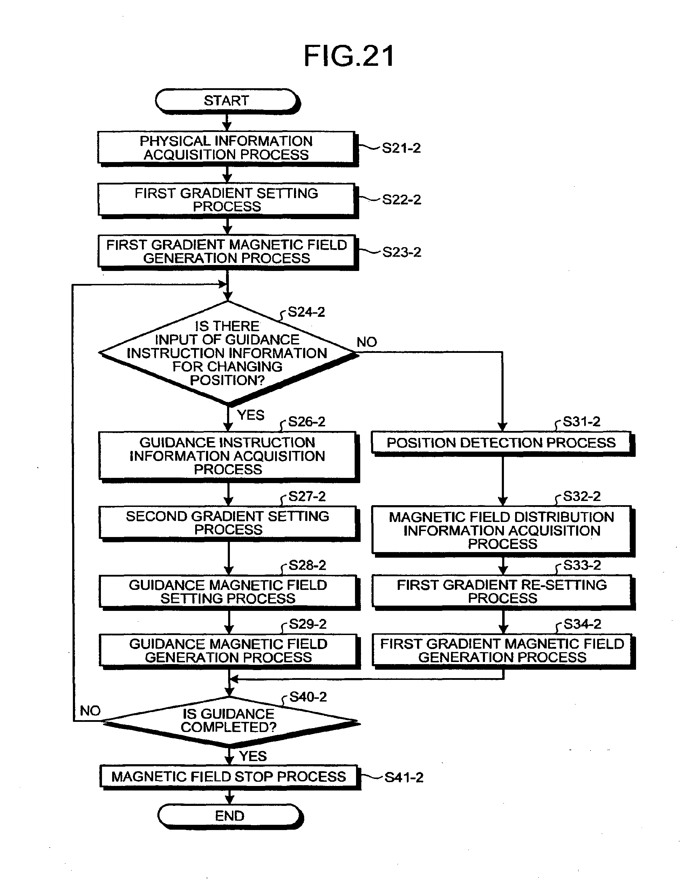

Next, a control process when the magnetic guidance is performed by the control unit 204a illustrated in FIG. 20 will be described. FIG. 21 is a flowchart illustrating a processing procedure when the magnetic guidance is performed by the control unit 204a illustrated in FIG. 20.

As illustrated in FIG. 21, the first magnetic field gradient setting unit 246a performs the physical information acquisition process (step S21-2), the first gradient setting process (step S22-2), and the first gradient magnetic field generation process (step S23-2) in the same processing procedure as that of steps S21 to S23 illustrated in FIG. 18 in order to obtain the first magnetic field gradient. Next, the magnetic field controller 245a determines whether or not there is an input of the guidance instruction information for changing the position of the capsule endoscope 210 by the operation input unit 7 (step S24-2). In the physical information acquisition process, all physical information not necessarily has to be acquired, and at least the magnetic moment of the capsule endoscope 210 only has to be acquired.

If the magnetic field controller 245a determines that there is no input of the guidance instruction information for changing the position of the capsule endoscope 210 by the operation input unit 7 (step S24-2: No), the position detector 249a performs a position detection process for detecting the position of the capsule endoscope 210 (step S31-2) and outputs the detection result to the first magnetic field gradient setting unit 246a in order to fine-tune the first magnetic field gradient so that the capsule endoscope 210 can be kept in a static state at a higher degree of accuracy. Next, the first magnetic field gradient setting unit 246a reads the magnetic field distribution information 208a from the storage unit 8 and performs a magnetic field distribution information acquisition process for acquiring magnetic field distribution information (step S32-2). The first magnetic field gradient setting unit 246a performs a first gradient re-setting process for re-setting the first magnetic field gradient on the basis of the position detection result of the position detector 249a and the acquired magnetic field distribution information 208a (step S33-2). In the first gradient re-setting process, the first magnetic field gradient setting unit 246a refers to the acquired magnetic field distribution information 208a and obtains a change value indicating how much the magnetic field gradient of the magnetic field generated by the magnetic field generator 2 changes at the position detected by the position detector 249a. Then the first magnetic field gradient setting unit 246a changes and re-sets the set first magnetic field gradient according to the obtained change value. Thereafter, the magnetic field controller 245a performs the first gradient magnetic field generation process for controlling the magnetic field generator 2 to apply a uniform gradient magnetic field having the first magnetic field gradient re-set by the first magnetic field gradient setting unit 246a (step S34-2).

On the other hand, if the magnetic field controller 245a determines that there is an input of the guidance instruction information for changing the position of the capsule endoscope 210 by the operation input unit 7 (step S24-2: Yes), in the same processing procedure as that of steps S6 to S9 illustrated in FIG. 8, the guidance instruction information acquisition process (step S26-2) and the second gradient setting process (step S27-2) are performed by the second magnetic field gradient setting unit 47, the guidance magnetic field setting process (step S28-2) is performed by the magnetic field setting unit 48, and the guidance magnetic field generation process (step S29-2) is performed by the magnetic field controller 245a. Next, in the same manner as in steps S10 and S11 illustrated in FIG. 8, the guidance completion determination process (step S40-2) and the magnetic field stop process (step S41-2) are performed by the magnetic field controller 245a.

Although not illustrated in FIG. 21, if the magnetic field controller 245a determines that there is an input of the guidance instruction information for changing the posture of the capsule endoscope 210 from the operation input unit 7, the magnetic field setting unit 48 changes the orientation of the magnetic field generated according to the inputted guidance instruction information.

As described above, in the first modified example of the second embodiment, if the guidance instruction information for changing the position of the capsule endoscope 210 is not inputted from the operation input unit 7, the first magnetic field gradient is automatically re-set according to the position of the capsule endoscope 210, so that it is possible to keep the capsule endoscope in a static state at a higher degree of accuracy regardless of the position of the capsule endoscope 210. Therefore, reflectiveness of an input into the operation input unit 7 to the magnetic guidance can be enhanced.

### Second Modified Example of the Second Embodiment

### (Modification 2 of Second Embodiment)

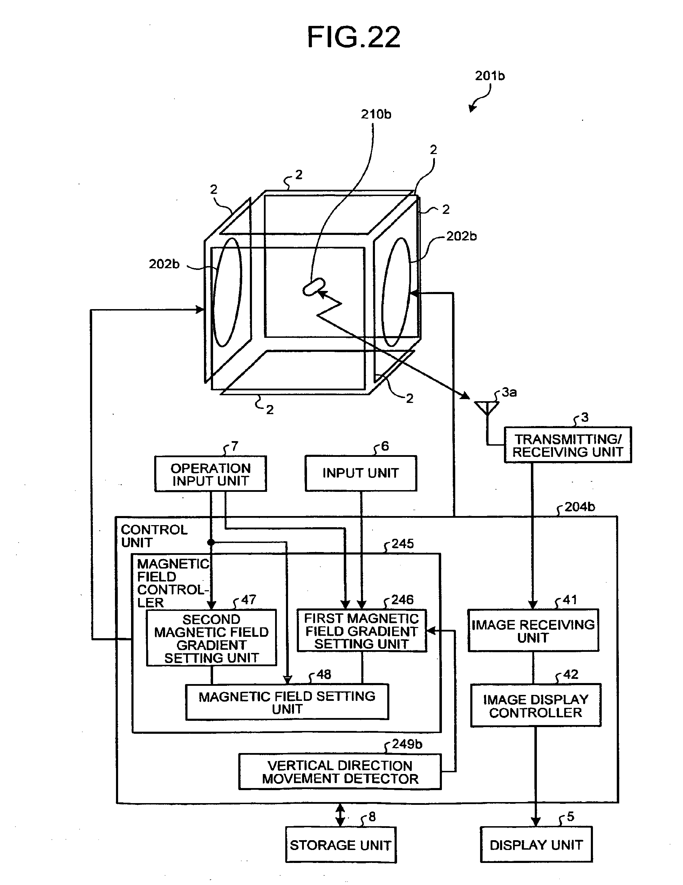

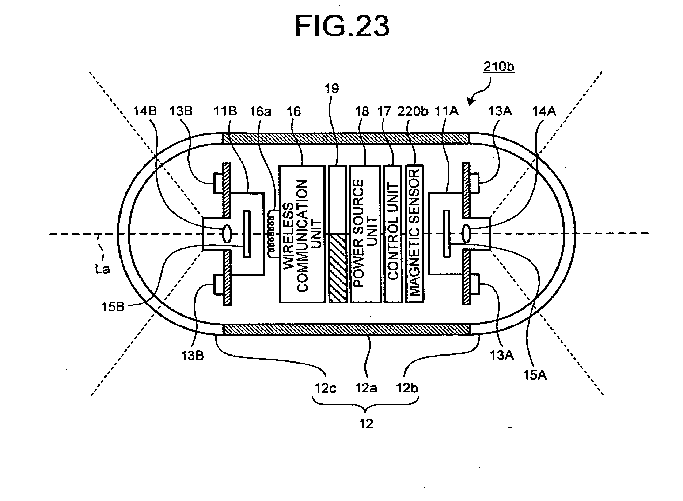

Next, a second modified example of the second embodiment will be described. FIG. 22 is a schematic diagram illustrating an entire configuration of a capsule medical device guidance system according to the second modified example of the second embodiment. As illustrated in FIG. 22, a capsule medical device guidance system 201b according to the second modified example of the second embodiment uses a capsule endoscope 210b which further includes a magnetic sensor 220b for detecting an alternating current magnetic field as illustrated in FIG. 23 instead of the capsule endoscope 10 illustrated in FIG. 2. Compared with the second embodiment, the capsule medical device guidance system 201b has a configuration including a control unit 204b instead of the control unit 204 and a position detection magnetic field generation unit 202b for generating a position detection alternating current magnetic field instead of the magnetic field detector 202. Compared with the control unit 204 illustrated in FIG. 16, the control unit 204b includes a vertical direction movement detector 249b instead of the vertical direction movement detector 249. The control unit 204b controls a magnetic field generation process for the position detection by the position detection magnetic field generation unit 202b.

In the capsule endoscope 210b, the magnetic sensor 220b detects the alternating current magnetic field generated by the position detection magnetic field generation unit 202b. The wireless communication unit 16 transmits the detection result of the magnetic sensor 220b to the transmitting/receiving unit 3.

The vertical direction movement detector 249b detects the vertical movement of the capsule endoscope 210b on the basis of the detection result of the magnetic sensor 220b outputted from the transmitting/receiving unit 3 and detects whether or not the capsule endoscope 210b moves in the vertical direction. For example, the vertical direction movement detector 249b continuously calculates the position coordinates and the orientation vector of the capsule endoscope 210b in three-dimensional space on the basis of the continuous detection results of the magnetic sensor 220b.

In the same manner as in the second embodiment, the first magnetic field gradient setting unit 246 re-sets the first magnetic field gradient obtained based on the physical parameter of the capsule endoscope 210b and the physical parameter of the liquid on the basis of the detection result of the vertical direction movement detector 249b.

Also in the capsule medical device guidance system 201b in which the magnetic sensor 220b is provided inside the capsule endoscope 210b and the position of the capsule endoscope 210b is detected by generating a position detection magnetic field from outside the capsule endoscope 210b, it is possible to automatically fine-tune the first magnetic field gradient until the vertical movement of the capsule endoscope 210 stops by performing each processing procedure illustrated in FIG. 18 or FIG. 19. Also in the capsule medical device guidance system 201b, it is possible to automatically re-set the first magnetic field gradient according to the position of the capsule endoscope 210b by causing the storage unit 8 to store the magnetic field distribution information 208a and performing each processing procedure illustrated in FIG. 21.

### Third Modified Example of the Second Embodiment

### (Modification 3 of Second Embodiment)

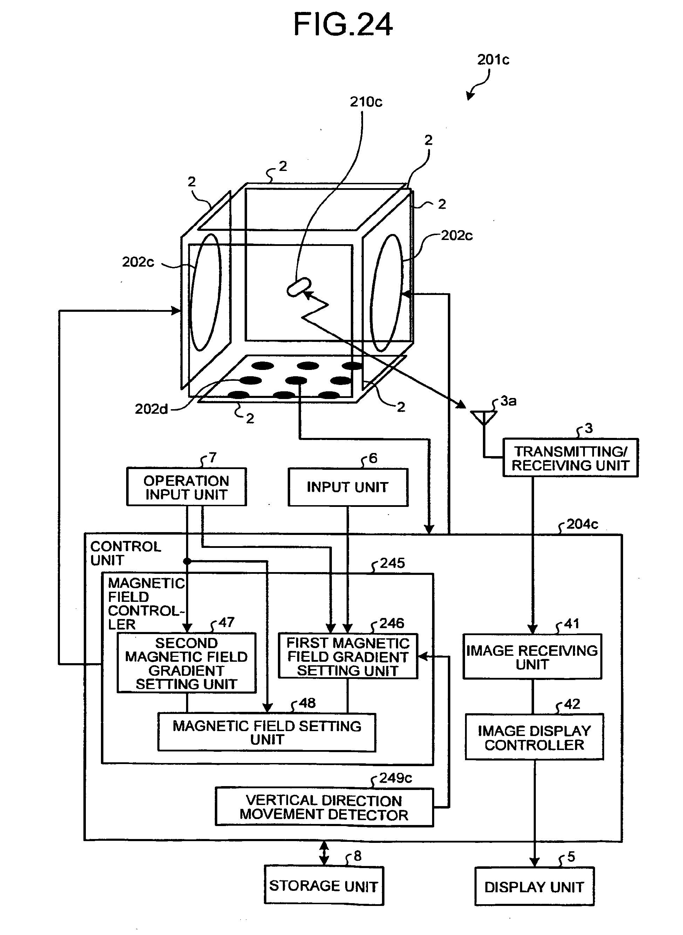

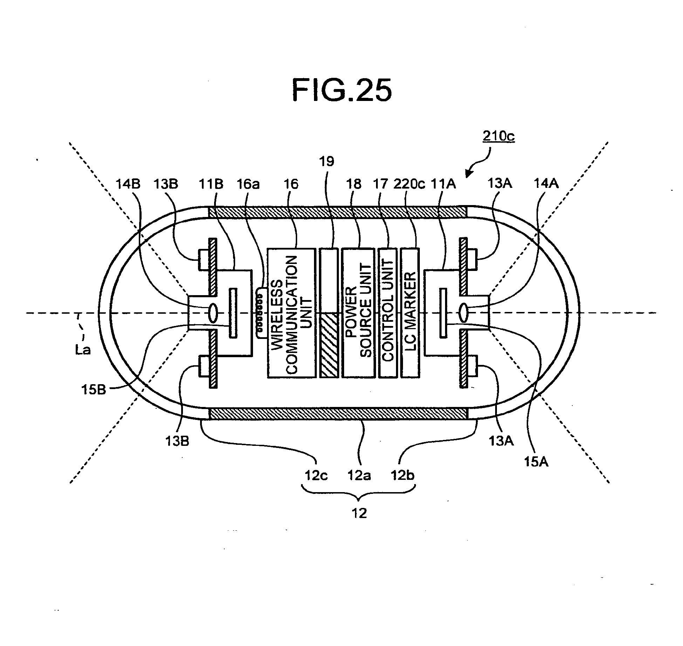

Next, a third modified example of the second embodiment will be described. FIG. 24 is a schematic diagram illustrating an entire configuration of a capsule medical device guidance system according to the third modified example of the second embodiment. As illustrated in FIG. 24, a capsule medical device guidance system 201c according to the third modified example of the second embodiment uses a capsule endoscope 210c which further includes an LC marker 220c that resonates with an alternating current magnetic field from the outside and generates a resonant magnetic field as illustrated in FIG. 25 instead of the capsule endoscope 10 illustrated in FIG. 2. Compared with the second embodiment, the capsule medical device guidance system 201c has a configuration including a control unit 204c instead of the control unit 204 and a position detection magnetic field generation unit 202c and a plurality of magnetic field detectors 202d that detects the resonant magnetic field generated by the LC marker 220c instead of the magnetic field detectors 202. Compared with the control unit 204 illustrated in FIG. 16, the control unit 204c includes a vertical direction movement detector 249c instead of the vertical direction movement detector 249. The control unit 204c controls a magnetic field generation process for the position detection by the position detection magnetic field generation unit 202c.

The vertical direction movement detector 249c detects the vertical movement of the capsule endoscope 210c on the basis of the detection results outputted from the magnetic field detectors 202d and determines whether or not the capsule endoscope 210c moves in the vertical direction. The vertical direction movement detector 249c continuously obtains a difference value between a detection value of the resonant magnetic field generated by the LC marker 220c at the magnetic field detectors 202d when the capsule endoscope is not located within a detection range and a detection value of the resonant magnetic field generated by the LC marker 220c at the magnetic field detectors 202d when the capsule endoscope is located within the detection range, and continuously calculates the position coordinates and the orientation vector of the capsule endoscope 210c in three-dimensional space on the basis of the difference values.

In the same manner as in the second embodiment, the first magnetic field gradient setting unit 246 re-sets the first magnetic field gradient obtained based on the physical parameter of the capsule endoscope 210c and the physical parameter of the liquid on the basis of the detection result of the vertical direction movement detector 249c.

Also in the capsule medical device guidance system 201c in which the LC marker 220c is provided inside the capsule endoscope 210c and the position of the capsule endoscope 210c is detected by detecting the resonant magnetic field of the LC marker 220c that resonates with the alternating current magnetic field from outside the capsule endoscope 210c, it is possible to automatically fine-tune the first magnetic field gradient until the vertical movement of the capsule endoscope 210c stops by performing each processing procedure illustrated in FIG. 18 or FIG. 19. Also in the capsule medical device guidance system 201c, it is possible to automatically re-set the first magnetic field gradient according to the position of the capsule endoscope 210c by causing the storage unit 8 to store the magnetic field distribution information 208a and performing each processing procedure illustrated in FIG. 21. Fourth Modified Example of the Second Embodiment

### (Modification 4 of Second Embodiment)

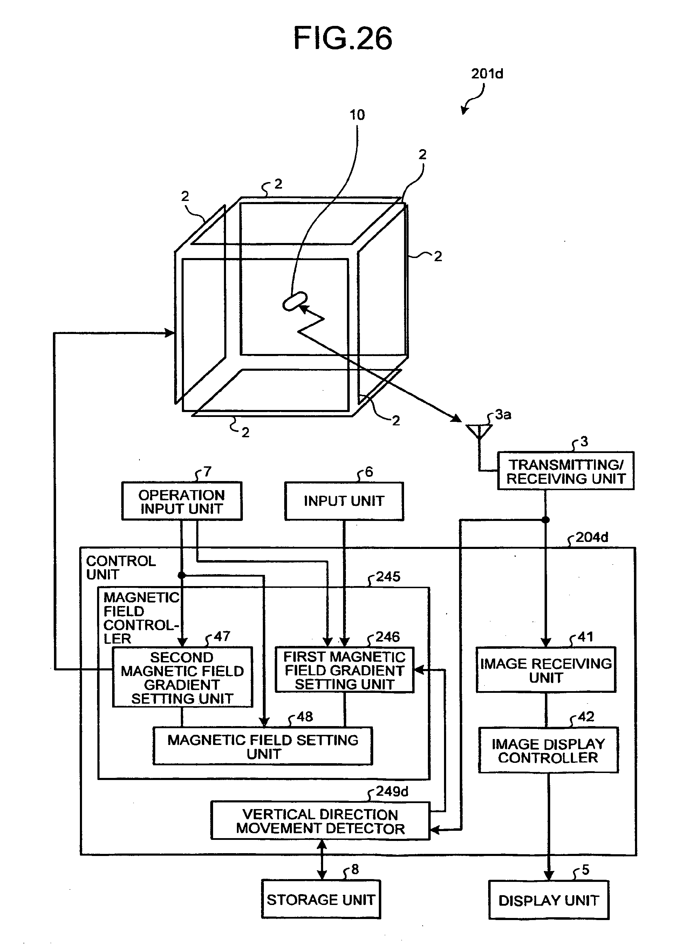

Next, a fourth modified example of the second embodiment will be described. FIG. 26 is a schematic diagram illustrating an entire configuration of a capsule medical device guidance system according to the fourth modified example of the second embodiment. As illustrated in FIG. 26, a capsule medical device guidance system 201d according to the fourth modified example of the second embodiment uses the capsule endoscope 10 illustrated in FIG. 2 and has a configuration in which a control unit 204d is included instead of the control unit 204 and the magnetic field detectors 202 are removed compared with the second embodiment. Compared with the control unit 204 illustrated in FIG. 16, the control unit 204d includes a vertical direction movement detector 249d instead of the vertical direction movement detector 249.

The vertical direction movement detector 249d continuously detects a receiving electric field strength of a signal transmitted from the capsule endoscope 10 at each antenna 3a on the basis of a receiving state of each antenna 3a of the transmitting/receiving unit 3, and detects the vertical movement of the capsule endoscope 10 from the variation of the receiving electric field strength of the signal.

In the same manner as in the second embodiment, the first magnetic field gradient setting unit 246 re-sets the first magnetic field gradient obtained based on the physical parameter of the capsule endoscope 10 and the physical parameter of the liquid on the basis of the detection result of the vertical direction movement detector 249d.

Also in the capsule medical device guidance system 201d which detects the vertical movement of the capsule endoscope 10 on the basis of the receiving electric field strength of the signal transmitted from the capsule endoscope 10, it is possible to automatically fine-tune the first magnetic field gradient until the vertical movement of the capsule endoscope 10 stops by performing each processing procedure illustrated in FIG. 18 or FIG. 19. Fifth Modified Example of the Second Embodiment

### (Modification 5 of Second Embodiment)

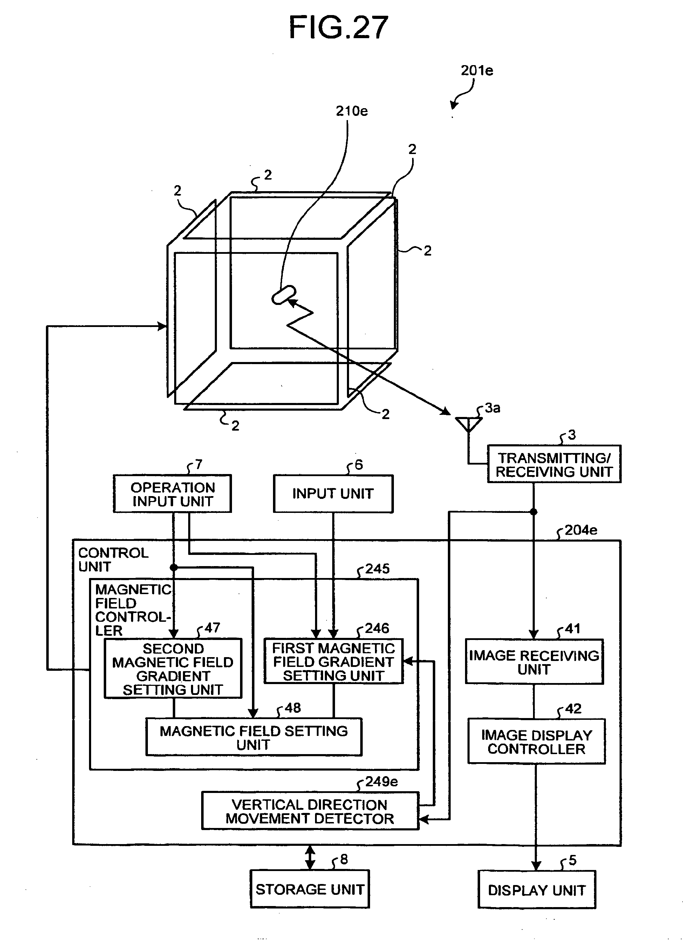

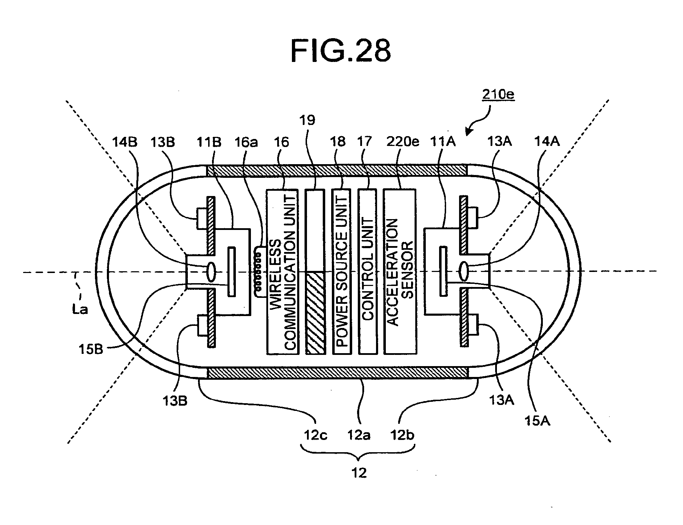

Next, a fifth modified example of the second embodiment will be described. FIG. 27 is a schematic diagram illustrating an entire configuration of a capsule medical device guidance system according to the fifth modified example of the second embodiment. As illustrated in FIG. 27, a capsule medical device guidance system 201e according to the fifth modified example of the second embodiment uses a capsule endoscope 210e, which further includes an acceleration sensor 220e as illustrated in FIG. 28 and transmits an output result of the acceleration sensor 220e to the transmitting/receiving unit 3, instead of the capsule endoscope 10 illustrated in FIG. 2. Compared with the second embodiment, the capsule medical device guidance system 201e has a configuration including a control unit 204e instead of the control unit 204. Compared with the control unit 204 illustrated in FIG. 16, the control unit 204e includes a vertical direction movement detector 249e instead of the vertical direction movement detector 249.

The vertical direction movement detector 249e adds up the output results of the acceleration sensor 220e transmitted from the capsule endoscope 210e, and thereby obtains a relative variation of the position of the capsule endoscope 210e, and detects the vertical movement of the capsule endoscope 210e.

In the same manner as in the second embodiment, the first magnetic field gradient setting unit 246 re-sets the first magnetic field gradient obtained based on the physical parameter of the capsule endoscope 210e and the physical parameter of the liquid on the basis of the detection result of the vertical direction movement detector 249e.

Also in the capsule medical device guidance system 201e in which the acceleration sensor 220e is provided inside the capsule endoscope 210e and the vertical movement of the capsule endoscope 210e is detected on the basis of the output result of the acceleration sensor 220e transmitted from the capsule endoscope 210e, it is possible to automatically fine-tune the first magnetic field gradient until the vertical movement of the capsule endoscope 210e stops by performing each processing procedure illustrated in FIG. 18 or FIG. 19.

### Third Embodiment

Next, a third embodiment will be described. In the third embodiment, a case will be described in which a first adjustment unit is provided and the first magnetic field gradient is fine-tuned by an adjustment operation of the first adjustment unit.

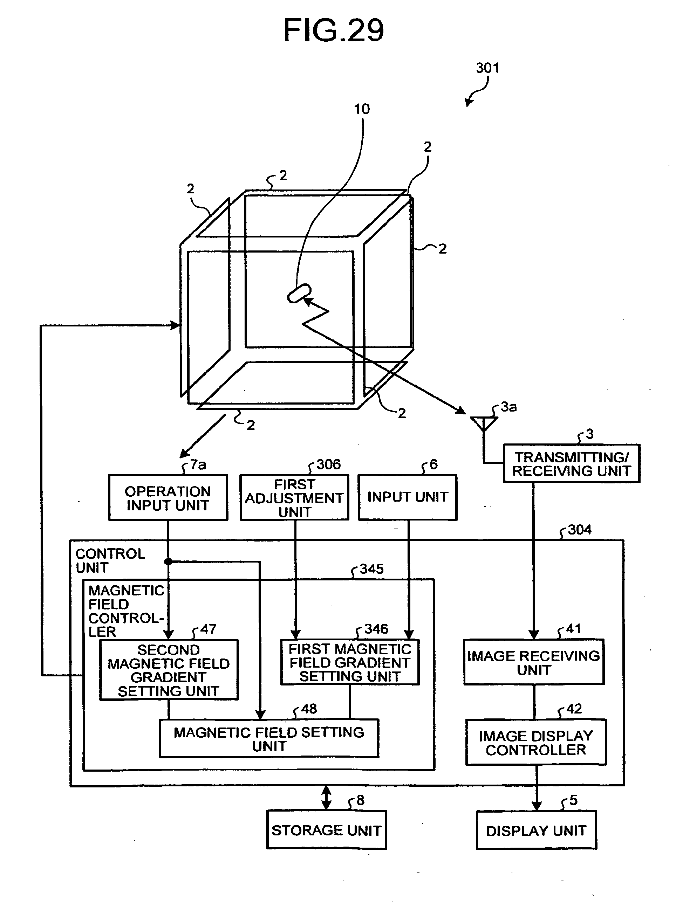

FIG. 29 is a schematic diagram illustrating an entire configuration of a capsule medical device guidance system according to the third embodiment. As illustrated in FIG. 29, a capsule medical device guidance system 301 according to the third embodiment has a configuration further including a first adjustment unit 306 compared with the capsule medical device guidance system 1 illustrated in FIG. 1. The capsule medical device guidance system 301 includes a control unit 304 instead of the control unit 4 illustrated in FIG. 1. The control unit 304 includes a magnetic field controller 345 instead of the magnetic field controller 45 illustrated in FIG. 1. The magnetic field controller 345 includes a first magnetic field gradient setting unit 346 instead of the first magnetic field gradient setting unit 46.

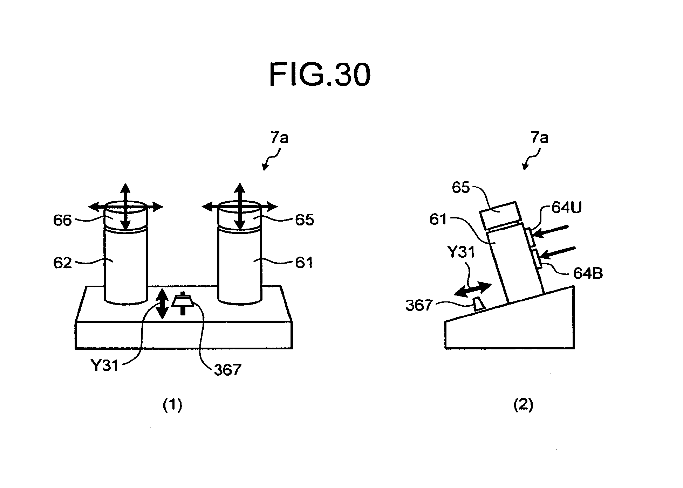

The first adjustment unit 306 has a function to input adjustment instruction information for instructing a gradient adjustment of the first magnetic field gradient into the first magnetic field gradient setting unit 346 of the control unit 304. For example, the first adjustment unit 306 includes a slide bar 367 provided between the joysticks 61 and 62 as illustrated in an operation input unit 7a of FIG. 30. The slide bar 367 can slide from front to rear as illustrated by the arrow Y31. For example, when the slide bar 367 is slid to the front, the first adjustment unit 306 inputs the adjustment instruction information for instructing to adjust the gradient of the first magnetic field gradient so that the vertical axis downward magnetic attracting force increases larger than the current setting according to the amount of slide to the control unit 304. On the other hand, when the slide bar 367 is slid to the rear, the first adjustment unit 306 inputs the adjustment instruction information for instructing to adjust the gradient of the first magnetic field gradient so that the vertical axis upward magnetic attracting force increases larger than the current setting according to the amount of slide to the control unit 304. The first adjustment unit 306 is not limited to a slide type one such as the slide bar 367 illustrated in FIG. 30, and for example, may be a pedal type one, a dial type one, and a type that increases and decreases the gradient by using two buttons.

The adjustment gradient adjusted by the first adjustment unit 306 is desired to be maintained after the adjustment. The adjustment means is desired to be one in which the adjustment result is maintained (a slide type one, a dial type one, a type that increases and decreases the gradient by using buttons, and the like).

The first magnetic field gradient setting unit 346 obtains the first magnetic field gradient which is a magnetic field gradient capable of keeping the capsule endoscope 10 in a static state on the basis of the adjustment instruction information inputted by the first adjustment unit 306 along with the physical parameter of the capsule endoscope 10 and the physical parameter of the liquid. The first magnetic field gradient setting unit 346 further fine-tunes and re-sets the first magnetic field gradient obtained based on the physical parameter of the capsule endoscope 10 and the physical parameter of the liquid on the basis of the adjustment instruction information inputted by the first adjustment unit 306.

In the third embodiment, a user slides the slide bar 367 while viewing a biological image captured by the capsule endoscope 10 displayed on the display unit 5. When adjusting the first magnetic field gradient, the user does not operate the joysticks 61 and 62, the up button 64U, and the down button 64B. The gradient of the first magnetic field gradient is adjusted by the first magnetic field gradient setting unit 346 according to the amount of slide of the slide bar 367. Next, when the user determines that the movement of the biological image stops, the user releases his or her hand from the slide bar 367, and ends the operation of the slide bar 367. As a result, the magnetic field gradient adjusted according to the adjustment instruction information inputted last time is set as the first magnetic field gradient.

Also in the case in which the first magnetic field gradient can be fine-tuned by an operation of the first adjustment unit 306 by a user as described in the third embodiment, the capsule endoscope can be more reliably kept in a static state.

Further, in the third embodiment, the set magnetic field gradient is maintained after the adjustment performed by the first adjustment unit 306, so that it is possible to guide the capsule endoscope 10 in an intended direction only by inputting an operation in a direction to which the capsule endoscope 10 is guided. Therefore, the operability can be improved.

### Fourth Embodiment

### (Fourth Embodiment)

Next, a fourth embodiment will be described. In the fourth embodiment, a case will be described in which a second adjustment unit is provided in addition to the first adjustment unit and a range width of the second magnetic field gradient is adjusted by an adjustment operation of the second adjustment unit.

FIG. 31 is a schematic diagram illustrating an entire configuration of a capsule medical device guidance system according to the fourth embodiment. As illustrated in FIG. 31, a capsule medical device guidance system 401 according to the fourth embodiment has a configuration further including a first adjustment unit 4061 and a second adjustment unit 4062 compared with the capsule medical device guidance system 1 illustrated in FIG. 1. The capsule medical device guidance system 1 includes a control unit 404 instead of the control unit 4 illustrated in FIG. 1. The control unit 404 includes a magnetic field controller 445 including a first magnetic field gradient setting unit 446, a second magnetic field gradient setting unit 447, and the magnetic field setting unit 48 instead of the magnetic field controller 45 illustrated in FIG. 1.

The first adjustment unit 4061 has a function to input first adjustment instruction information for instructing a gradient adjustment of the first magnetic field gradient to the first magnetic field gradient setting unit 446 of the control unit 404. For example, the first adjustment unit 4061 includes a first magnetic field gradient up button 4061U and a first magnetic field gradient down button 4061D provided between the joysticks 61 and 62 as illustrated in an operation input device 407 of FIG. 32. When the first magnetic field gradient up button 4061U is pressed once, the first magnetic field gradient up button 4061U inputs the first adjustment instruction information for instructing to adjust the first magnetic field gradient so that the magnetic attracting force becomes relatively larger than the current setting by a predetermined change amount in the vertically upward direction into the control unit 404. When the first magnetic field gradient down button 4061D is pressed once, the first magnetic field gradient down button 4061D inputs the first adjustment instruction information for instructing so that the magnetic attracting force becomes relatively larger than the current setting by a predetermined change amount in the vertically downward direction into the control unit 404. Hereinafter, the change generated when the first magnetic field gradient up button 4061U or the first magnetic field gradient down button 4061D is pressed once is referred to as a one level change.

When the first adjustment instruction information is inputted from the first adjustment unit 4061, the first magnetic field gradient setting unit 446 re-sets the first magnetic field gradient from the currently set first magnetic field gradient set based on the physical parameter of the capsule endoscope 10 and the physical parameter of the liquid so that the magnetic attracting force changes relatively in the vertically upward direction or the vertically downward direction according to the first adjustment instruction information inputted from the first adjustment unit 4061.

For example, when the first magnetic field gradient up button 4061U is pressed four times by a user, the first adjustment instruction information for instructing to adjust the first magnetic field gradient so that the magnetic attracting force becomes larger than the current setting by four levels in the vertically upward direction is inputted into the control unit 404. FIG. 33 is a diagram illustrating a relationship between each level (CL) of the first magnetic field gradient and the magnetic attracting force (F) generated by the magnetic field of each level of the first magnetic field gradient. For example, as illustrated in FIG. 33, when the first gradient level is set to level 2, the first gradient level is changed to level 6. Specifically, when a magnetic attracting force Fm1-2 corresponding to level 2 is a vertically upward magnetic attracting force as illustrated in FIG. 33, a generation of a vertically upward magnetic attracting force Fm1-6 having a magnitude corresponding to level 6 that is four-level higher than level 2 as illustrated by the arrow Y41 is instructed. Therefore, the first magnetic field gradient setting unit 446 sets a magnetic field gradient of a magnetic field that generates the magnetic attracting force Fm1-6 as the first magnetic field gradient, and the magnetic field setting unit 48 causes the magnetic field generator 2 to generate a magnetic field having the first magnetic field gradient corresponding to the level 6.

As a result, as illustrated in FIG. 33, even when a resultant force of a resultant force Ffg of the buoyancy and the gravity force of the capsule endoscope 10 and the vertical magnetic attracting force Fm1-2 is not substantially zero, it is possible to make the resultant force of the gravity force, the buoyancy, and the magnetic attracting force substantially zero by the magnetic attracting force Fm1-6 which has substantially the same magnitude as that of the resultant force Ffg and which is directed in the opposite direction to the resultant force Ffg. As illustrated in FIG. 33, if the first magnetic field gradient is set so that the magnetic attracting force is directed in the vertical axis upward direction, when the first magnetic field gradient up button 4061U is pressed once, the magnetic attracting force becomes larger by one level in the vertical axis upward direction. If the first magnetic field gradient is set so that the magnetic attracting force is directed in the vertical axis downward direction, when the first magnetic field gradient up button 4061U is pressed once, the magnetic attracting force in the vertical axis downward direction becomes smaller by one level. Or, the direction of the magnetic attracting force is changed from vertically downward to vertically upward. If the first magnetic field gradient is set so that the magnetic attracting force is directed in the vertical axis upward direction, when the first magnetic field gradient down button 4061D is pressed once, the magnetic attracting force in the vertical axis upward direction becomes smaller by one level. Or, the direction of the magnetic attracting force is changed from vertically upward to vertically downward. If the first magnetic field gradient is set so that the magnetic attracting force is directed in the vertical axis downward direction, when the first magnetic field gradient down button 4061D is pressed once, the magnetic attracting force becomes larger by one level in the vertical axis downward direction. The number of the levels corresponding to the first gradient is 11 levels from level 0 to level 10, for example. In the case of level 0, a magnetic field gradient of a magnetic field for generating a vertically downward magnetic attracting force by a maximum force within a predetermined allowable generation range of magnetic attracting force is set as the first magnetic field gradient. In the case of level 11, a magnetic field gradient of a magnetic field for generating a vertically upward magnetic attracting force by a maximum force within a predetermined allowable generation range of magnetic attracting force is set as the first magnetic field gradient.

The second adjustment unit 4062 inputs instruction information for instructing a gradient adjustment of the second magnetic field gradient into the second magnetic field gradient setting unit 447. An allowable setting range of the second magnetic field gradient can be changed to small or large, and the second adjustment unit 4062 inputs range width instruction information for instructing a range width of the second magnetic field gradient into the second magnetic field gradient setting unit 447.

For example, the second adjustment unit 4062 includes a second magnetic field gradient up button 4062U and a second magnetic field gradient down button 4062D provided between the joysticks 61 and 62 as illustrated in an operation input unit 407 of FIG. 32. When the second magnetic field gradient up button 4062U is pressed once, the second magnetic field gradient up button 4062U inputs the second adjustment instruction information for instructing to adjust the allowable setting range of the second magnetic field gradient so that the magnitude of the magnetic attracting force corresponding to the second magnetic field gradient generated according to an amount of operation of the joysticks 61 and 62 increases by a predetermined ratio into the control unit 404. When the second magnetic field gradient down button 4062D is pressed once, the second magnetic field gradient down button 4062D inputs the second adjustment instruction information for instructing to adjust the allowable setting range of the second magnetic field gradient so that the magnitude of the magnetic attracting force corresponding to the second magnetic field gradient generated according to an amount of operation of the joysticks 61 and 62 decreases by a predetermined ratio into the control unit 404. Hereinafter, the change of the width of the allowable setting range of the second magnetic field gradient when the second magnetic field gradient up button 4062U or the second magnetic field gradient down button 4062D is pressed once is referred to as a one level change.

The second magnetic field gradient setting unit 447 sets the second magnetic field gradient based on the second adjustment instruction information inputted by the second adjustment unit 4062. The second magnetic field gradient setting unit 447 defines a range of a magnetic field gradient, whose center value is the first magnetic field gradient set by the first magnetic field gradient setting unit 446 and which has a range width instructed by the range width instruction information inputted from the second adjustment unit 4062, as the allowable setting range of the second magnetic field gradient. The second magnetic field gradient setting unit 447 sets the second magnetic field gradient so that the second magnetic field gradient is included in the allowable setting range of the second magnetic field gradient. At this time, the second magnetic field gradient setting unit 447 sets the allowable setting range of the second magnetic field gradient so that the allowable setting range does not exceed a predetermined allowable generation range of magnetic attracting force in the device. When a sum of the first magnetic field gradient and the second magnetic field gradient in the vertical direction exceeds a predetermined allowable generation range of magnetic attracting force in the vertical direction, the magnetic field setting unit 48 controls the magnetic field generator 2 to apply a magnetic field having a magnetic field gradient of an upper limit value or a lower limit value of the range instead of the sum of the first magnetic field gradient and the second magnetic field gradient in the vertical direction.

Here, as an example, a case will be described in which the magnetic attracting force corresponding to the second magnetic field gradient, that is, the magnetic attracting force for guiding the capsule endoscope 10, which has a magnitude proportional to the amount of operation of the joysticks 61 and 62, is generated. For example, the current setting is set so that the magnetic attracting force corresponding to the second magnetic field gradient is generated corresponding to the amount of operation of the joysticks 61 and 62 in accordance with the relationship indicated by the straight line Lv in FIG. 34. FIG. 34 illustrates relationships corresponding to an amount of operation in one direction among a plurality of directions in which the joysticks 61 and 62 can be operated.

When the second magnetic field gradient up button 4062U is pressed once by a user, as illustrated by the arrow Y43, it is set so that the magnetic attracting force corresponding to the second magnetic field gradient is generated in accordance with a relationship indicated by the straight line Lvu1 having a slope one level larger than that of the straight line Lv. As a result, a value range of the magnetic attracting force generated corresponding to the amount of operation of the joysticks 61 and 62 is widened by one level, so that, even if the amount of operation of the joysticks is the same amount of operation Hj, a magnetic attracting force Fvu1 larger than a magnetic attracting force Fv, which is the magnetic attracting force before the second magnetic field gradient up button 4062U is pressed, is generated. Further, when the second magnetic field gradient up button 4062U is pressed once more in this state, as illustrated by the arrow Y44, it is set so that the magnetic attracting force corresponding to the second magnetic field gradient is generated in accordance with a relationship indicated by the straight line Lvu2 having a slope one level larger than that of the straight line Lvu1. As a result, a value range of the magnetic attracting force generated corresponding to the amount of operation of the joysticks 61 and 62 is further widened by one level, so that, when the amount of operation of the joysticks is Hj, a magnetic attracting force Fvu2 (> Fvu1) is generated.

When the second magnetic field gradient down button 4062D is pressed once by a user, as illustrated by the arrow Y45, it is set so that the magnetic attracting force corresponding to the second magnetic field gradient is generated in accordance with a relationship indicated by the straight line Lvd1 having a slope one level smaller than that of the straight line Lv. As a result, a value range of the magnetic attracting force generated corresponding to the amount of operation of the joysticks 61 and 62 is narrowed by one level, so that, even if the amount of operation of the joysticks is the same amount of operation Hj, a magnetic attracting force Fvd1 smaller than a magnetic attracting force Fv, which is the magnetic attracting force before the second magnetic field gradient down button 4062D is pressed, is generated. Further, when the second magnetic field gradient down button 4062D is pressed once more in this state, as illustrated by the arrow Y46, it is set so that the magnetic attracting force corresponding to the second magnetic field gradient is generated in accordance with a relationship indicated by the straight line Lvd2 having a slope one level smaller than that of the straight line Lvd1. As a result, a value range of the magnetic attracting force generated corresponding to the amount of operation of the joysticks 61 and 62 is further narrowed by one level, so that, when the amount of operation of the joysticks is Hj, a magnetic attracting force Fvd2 (< Fvd1) is generated.

At this time, in a menu screen Sa (see FIG. 35) displayed on the display unit 5, in the center lower area S41, a first block Bc displayed so that each level of the magnetic attracting force corresponding to the first magnetic field gradient (in FIG. 35, corresponding to "Calibration force") is known is displayed. For example, when the level of the first magnetic field gradient is 6, the blocks up to level 6 are displayed in a bright color, and the blocks after the level 6 are displayed in a dark color. The user can recognize the level of the magnetic attracting force corresponding to the first magnetic field gradient applied to keep the capsule endoscope 10 in a static state at this time by checking the first block Bc.

In the area S41, a second block Bv is displayed in which the range width of the magnetic attracting force that can be generated corresponding to the second magnetic field gradient (in FIG. 5, corresponding to "Vertical force") is displayed. Here, the levels of the magnetic attracting force that can be generated corresponding to the second magnetic field gradient are set at the same pitch as that of the levels of the magnetic attracting force corresponding to the first magnetic field gradient.

For example, the magnetic attracting force corresponding to the first magnetic field gradient is set to level 6 and the range width of the magnetic attracting force that can be generated corresponding to the second magnetic field gradient is set to a range of levels 3 to 9, whose center is the level 6 and which includes 3 levels just above the level 6 and 3 levels just below the level 6. In this case, as illustrated in FIG. 35, in the second block Bv, the blocks of levels 3 to 9 are displayed in a bright color and the blocks of levels 1 to 3 and level 10 are displayed in a dark color. In this example, the levels 3 to 5 corresponds to a range in which the magnetic attracting force is generated in the downward direction and the levels 7 to 9 corresponds to a range in which the magnetic attracting force is generated in the upward direction.

In this state, when the second magnetic field gradient up button 4062U is pressed once, the range width of the magnetic attracting force that can be generated corresponding to the second magnetic field gradient is widened both upward and downward by one level to be set to levels 2 to 10. The display of the second block Bv in the display menu Sa is also changed and the levels 2 to 10 are displayed in a bright color. Further, when the second magnetic field gradient up button 4062U is pressed once more, the range width of the magnetic attracting force that can be generated corresponding to the second magnetic field gradient is widened by one level. In this state, the level 10 is the maximum level, so that the range width of the magnetic attracting force that can be generated corresponding to the second magnetic field gradient is set to levels from 1 to 10 that is the maximum level. The display of the second block Bv in the display menu Sa is also changed and the levels 1 to 10 are displayed in a bright color.

On the other hand, when the second magnetic field gradient down button 4062D instead of the second magnetic field gradient up button 4062U is pressed once, the range width of the magnetic attracting force that can be generated corresponding to the second magnetic field gradient is narrowed both upward and downward by one level to levels 4 to 8. The display of the second block Bv in the display menu Sa is also changed and the levels 4 to 8 are displayed in a bright color. Further, when the second magnetic field gradient down button 4062D is pressed once more, the range width of the magnetic attracting force that can be generated corresponding to the second magnetic field gradient is further narrowed both upward and downward by one level to levels 5 to 7. The user can recognize the range width of the magnetic attracting force corresponding to the second magnetic field gradient, which can be operated at this time, by checking the second block Bc.

In this way, in the fourth embodiment, the range width of the second magnetic field gradient can be adjusted by an operation of the second adjustment unit 4062 by a user, and the allowable setting range of the second magnetic field is set by using the first magnetic field gradient as the center value. Therefore, in the fourth embodiment, the second magnetic field gradient corresponding to the amount of operation of the joystick is set by using the first magnetic field gradient as the center value thereof, so that the magnitude of the generated magnetic attracting force is the same between the upward operation of the joystick and the downward operation of the joystick. Therefore, according to the fourth embodiment, the amounts of upward and downward operations of the joystick are evenly reflected on the movement of the capsule endoscope 10 without a difference between upward and downward operations, so that the operational feeling of the user can be further matched to the movement of the capsule endoscope.

In the fourth embodiment, the control unit 404 may display the levels corresponding to the magnitude of the magnetic attracting force actually generated corresponding to the amount of operation of the joystick in a color different from that of the range width of the magnetic attracting force that can be generated corresponding to the second magnetic field gradient in the block Bv in the display menu Sa. For example, when the range width of the magnetic attracting force that can be generated corresponding to the second magnetic field gradient is set to a range of levels 3 to 9, in the second block Bv, the blocks of levels 3 to 9 may be displayed in white color, and when the level corresponding to the magnitude of the magnetic attracting force actually generated corresponding to the amount of operation of the joystick is 8, a predetermined portion between the level 6 and the level 8 (a portion with triangular-shaped marks in the example illustrated in FIG. 35) may be displayed in red color. In this way, the operator can check in real time the level of the magnetic attracting force corresponding to the second magnetic field gradient actually generated corresponding to the amount of operation of the joystick, so that the operability may be further improved.

Although the second magnetic field gradient setting unit 447 is described by using the example in which the magnetic attracting force corresponding to the second magnetic field gradient is generated proportional to the amount of operation of the joysticks 61 and 62, it is not limited to this, and the second magnetic field gradient may be set in the allowable setting range regardless of the amount of operation of the joysticks. For example, when the joystick is operated in the upward direction, the second magnetic field gradient setting unit 447 may set the maximum value of the allowable setting range of the second magnetic field gradient (in FIG. 35, the magnetic field gradient that generates a magnetic attracting force corresponding to level 9) as the second magnetic field gradient corresponding to the vertical axis upward magnetic attracting force. On the other hand, when the joystick is operated in the downward direction, the second magnetic field gradient setting unit 447 may set the minimum value of the allowable setting range of the second magnetic field gradient (in FIG. 35, the magnetic field gradient that generates a magnetic attracting force corresponding to level 3) as the second magnetic field gradient corresponding to the vertical axis downward magnetic attracting force.

### Fifth Embodiment

### (Fifth Embodiment)

Next, a fifth embodiment will be described. In the fifth embodiment, a case will be described in which a water surface mode in which the capsule endoscope is located on the water surface and a water bottom mode in which the capsule endoscope is located on the water bottom are set as a guidance mode of the capsule endoscope.

FIG. 36 is a schematic diagram illustrating an entire configuration of a capsule medical device guidance system according to the fifth embodiment. As illustrated in FIG. 36, a capsule medical device guidance system 501 according to the fifth embodiment has a configuration that includes a second adjustment unit 5062 instead of the second adjustment unit 4062 and further includes a mode setting unit 5063 compared with the capsule medical device guidance system 401 illustrated in FIG. 31. The capsule medical device guidance system 501 includes a control unit 504 instead of the control unit 404 illustrated in FIG. 31. The control unit 504 includes a magnetic field controller 545 including the first magnetic field gradient setting unit 446, a second magnetic field gradient setting unit 547, and the magnetic field setting unit 48 instead of the magnetic field controller 445 illustrated in FIG. 31.

In the fifth embodiment, as a guidance mode of the capsule endoscope 10, as illustrated in FIG. 37, a liquid surface mode in which the capsule endoscope 10 is located on a liquid surface Ws which is an upper boundary of a liquid W and a liquid bottom mode in which the capsule endoscope 10 is located on the liquid bottom (for example, a lower surface of a stomach wall St) which is a lower boundary of the liquid W are set. The liquid surface mode includes a case in which the capsule endoscope 10 is in contact with an upper surface of an inner wall of an organ (for example, an upper surface of the stomach wall St).

The mode setting unit 5063 inputs liquid surface mode instruction information for disposing the capsule endoscope 10 on the liquid surface Ws and liquid surface mode instruction information for disposing the capsule endoscope 10 on the liquid bottom as the guidance instruction information. For example, as illustrated in an operation input device 507 of FIG. 38, the mode setting unit 5063 includes a liquid surface mode button 5063S and a liquid bottom mode button 5063B provided on the right side of the joystick 61.

When the liquid surface mode button 5063S is pressed, the liquid surface mode instruction information is inputted into the control unit 504. When the liquid surface mode instruction information is inputted, the second magnetic field gradient setting unit 547 sets a magnetic field gradient of a magnetic field for generating a vertically upward magnetic attracting force by a maximum force within a predetermined allowable generation range of magnetic attracting force in the device as the second magnetic field gradient. For example, as illustrated in an area S42 of a menu screen Sb, for example, when the predetermined allowable generation range of magnetic attracting force is divided into 11 levels from level 0 to level 10, the second magnetic field gradient is set to generate a magnetic attracting force of level 10.

When the liquid bottom mode button 5063B is pressed, the liquid bottom mode instruction information is inputted into the control unit 504. In this case, when the liquid bottom mode instruction information is inputted, the second magnetic field gradient setting unit 547 sets a magnetic field gradient of a magnetic field for generating a vertically downward magnetic attracting force by the maximum force within the predetermined allowable generation range of magnetic attracting force in the device as the second magnetic field gradient. For example, the second magnetic field gradient is set to generate a magnetic attracting force of level 0 among the levels 0 to 10 in FIG. 39.

Further, in the fifth embodiment, a jumping mode for separating the capsule endoscope 10 disposed on the boundary of the liquid from the boundary in the liquid surface mode and the liquid bottom mode is set. For example, as illustrated in the operation input device 507 of FIG. 38, a jumping mode button 5063J is provided on the left side of the joystick 62. When the jumping mode button 5063J is pressed, jumping mode instruction information is inputted into the control unit 504.

When the jumping mode instruction information is inputted, the second magnetic field gradient setting unit 547 sets a magnetic field gradient of a magnetic field for generating a magnetic attracting force for separating the capsule endoscope 10 disposed on the boundary of the liquid from the boundary.

If the jumping mode button 5063J is pressed when the liquid surface mode is selected, the second magnetic field gradient setting unit 547 sets a magnetic field gradient of a magnetic field for generating a magnetic attracting force whose force in the vertically downward direction is relatively larger than the magnetic attracting force generated corresponding to the magnetic field having the first gradient as the second magnetic field gradient. While the jumping mode button 5063J is pressed when the liquid surface mode is selected, the magnetic field generator 2 generates, for example, a magnetic field for generating a vertically downward magnetic attracting force Fmj21 illustrated in FIG. 40 as a magnetic field corresponding to the second magnetic field gradient. As a result, the capsule endoscope 10 located on the liquid surface Ws jumps downward from the liquid surface Ws as illustrated by the arrow Y51 and separates from the liquid surface Ws. Considering the trapping of the capsule endoscope 10 by the surface tension of the liquid surface Ws, a strong downward magnetic attracting force may be temporarily applied so that the capsule endoscope 10 can reliably jump downward.

If the jumping mode button 5063J is pressed when the liquid bottom mode is selected, the second magnetic field gradient setting unit 547 sets a magnetic field gradient of a magnetic field for generating a magnetic attracting force whose force in the vertically upward direction is relatively larger than the magnetic attracting force generated corresponding to the magnetic field having the first gradient as the second magnetic field gradient. While the jumping mode button 5063J is pressed when the liquid surface mode is selected, the magnetic field generator 2 generates, for example, a magnetic field for generating a vertically upward magnetic attracting force Fmj22 illustrated in FIG. 40 as a magnetic field corresponding to the second magnetic field gradient. As a result, the capsule endoscope 10 located on the liquid bottom jumps upward from the liquid bottom as illustrated by the arrow Y52 and separates from the stomach wall St which is the boundary of the liquid bottom.

The second adjustment unit 5062 inputs the range width instruction information for instructing the range width of the second magnetic field gradient when the jumping mode is selected into the second magnetic field gradient setting unit 547. In the same manner as in the fourth embodiment, the range width of the second magnetic field gradient when the jumping mode is selected is set by the second magnetic field gradient setting unit 547 so that the range width has the first magnetic field gradient set by the first magnetic field gradient setting unit 446 as the center value thereof and has the range width which is instructed in the range width instruction information inputted from the second adjustment unit 5062. In the same manner as in the fourth embodiment, the second magnetic field gradient setting unit 547 sets the allowable setting range of the second magnetic field gradient so that the allowable setting range does not exceed the predetermined allowable generation range of magnetic attracting force.

The second adjustment unit 5062 includes a second magnetic field gradient up button 5062U and a second magnetic field gradient down button 5062D provided between the joysticks 61 and 62 as illustrated in FIG. 38. When the second magnetic field gradient up button 5062U is pressed once, the second magnetic field gradient up button 5062U inputs second adjustment instruction information for instructing adjustment of the second magnetic field gradient so that the range width of the second magnetic field gradient when the jumping mode is selected is widened by one level into the control unit 504. When the second magnetic field gradient down button 5062D is pressed once, the second magnetic field gradient down button 5062D inputs second adjustment instruction information for instructing adjustment of the second magnetic field gradient so that the range width of the second magnetic field gradient when the jumping mode is selected is narrowed by one level into the control unit 504.

As illustrated in a display menu Sb displayed on the display unit 5 illustrated in FIG. 39, in an area S42, a third block Bj is displayed in which the range width of the magnetic attracting force that can be generated corresponding to the second magnetic field gradient when the jumping mode is selected (in FIG. 39, corresponding to "Jumping force") is displayed. For example, the magnetic attracting force corresponding to the first magnetic field gradient is set to level 6 and the range width of the magnetic attracting force when the jumping mode is selected is set to a range of levels 3 to 9, whose center is the level 6 and which includes 3 levels just above the level 6 and 3 levels just below the level 6. In this setting, when the jumping mode is selected in the liquid surface mode, the second magnetic field gradient setting unit 547 sets the vertical axis downward magnetic field gradient corresponding to level 3 so that a vertical axis downward magnetic attracting force is generated by a largest force within the range width as the second magnetic field gradient. On the other hand, when the jumping mode is selected in the liquid bottom mode, the second magnetic field gradient setting unit 547 sets the vertical axis upward magnetic field gradient corresponding to level 9 so that a vertical axis upward magnetic attracting force is generated by a largest force within the range width as the second magnetic field gradient.

In this state, when the second magnetic field gradient up button 5062U is pressed once, the range width of the magnetic attracting force when the jumping mode is selected is widened by one level to levels 2 to 10. The display of the third block Bj in the display menu Sb is also changed and the levels 2 to 10 are displayed in a bright color. Further, when the second magnetic field gradient up button 5062U is pressed once more, the range width of the magnetic attracting force that can be generated corresponding to the second magnetic field gradient is widened by one level to levels 1 to 10 that is the maximum level.

On the other hand, when the second magnetic field gradient down button 5062D instead of the second magnetic field gradient up button 5062U is pressed once, the range width of the magnetic attracting force when the jumping mode is selected is narrowed both upward and downward by one level to levels 4 to 8. The display of the third block Bj in the display menu Sb is also changed and the levels 4 to 8 are displayed in a bright color. Further, when the second magnetic field gradient down button 5062D is pressed once more, the range width of the magnetic attracting force that can be generated corresponding to the second magnetic field gradient is further narrowed both upward and downward by one level to levels 5 to 7. The user can recognize the range width of the magnetic attracting force in the jumping mode, which can be operated at this time, by checking the third block Bj.

In this way, in the fifth embodiment, the allowable setting range of the second magnetic field gradient when the jumping mode is selected is set by using the first magnetic field gradient as the center value. Therefore, in the fifth embodiment, when the jumping mode is selected, whichever of the liquid surface mode or the liquid bottom mode is selected, it is possible to cause the capsule endoscope 10 to jump and move upward or downward evenly, so that the operational feeling of the user can be further matched to the movement of the capsule endoscope.

In the fifth embodiment, the values in each level of "Calibration force" and "Jumping force" set when the capsule endoscope 10 is observed may be respectively stored in association with images. In this case, when an image is reproduced after the observation, the first block Bc and the third block Bj which display each level of the "Calibration force" and the "Jumping force" associated with the reproduced image may be displayed in a reproduction menu Sc (see FIG. 41) so that it is possible to know the level of the magnetic attracting force applied when the observation is performed. In an area S44 in the display menu Sc, an icon Ip for instructing reproduction of an image and an icon Is for temporarily stopping the reproduction are displayed.

The first to the fifth embodiments can be applied when the magnetic field generator 2 generates a peak magnetic field instead of the uniform gradient magnetic field. As illustrated by a peak magnetic field Mp in FIG. 42, the peak magnetic field is a magnetic field that has a peak of magnetic field strength in a direction perpendicular to the horizontal surface. The peak magnetic field can attract the permanent magnet 19 to the peak position of the magnetic field strength and trap the capsule endoscope 10. In other words, the peak magnetic field is a trapping magnetic field that attracts the permanent magnet 19 of the capsule endoscope 10 to an arbitrary position in the horizontal direction and traps the capsule endoscope 10. For example, the magnetic field generator 2 can move the capsule endoscope 10 from the position Pc1 to the position Pc2 as illustrated by the arrow Y4 by moving the peak position of the peak magnetic field Mp from the position Pc1 to the position Pc2 as illustrated by the arrow Y3 in FIG. 42.

The peak magnetic field is a magnetic field having the same magnetic field strength on a concentric circle around the magnetic field generator. As illustrated in FIG. 43, when the peak magnetic field is generated, arc-shaped isodynamic lines Le1 to Le5 around the magnetic field generator 2a are located within the guidance range. Therefore, the peak magnetic field is a magnetic field that generates a magnetic gradient in the vertical direction. When the peak magnetic field is generated, magnetic force lines Lg1 to Lg5 are perpendicular to the isodynamic lines Le1 to Le5.

The first magnetic field gradient setting units 46, 246, 246a, 346, and 446 obtain a vertical magnetic field gradient of a peak magnetic field that generates a vertical magnetic attracting force which makes a resultant force of the buoyancy of the capsule endoscope 10 and the gravity force and the vertical magnetic attracting force of the capsule endoscope 10 in the liquid substantially zero on the basis of the physical information such as the mass, the volume, and the magnetic moment of the capsule endoscopes 10, 210, 210b, 210c, and 210e and the density of the liquid W, and sets the obtained vertical magnetic field gradient as the first magnetic field gradient. When the guidance instruction information for changing the position of the capsule endoscope 10 is not inputted from the operation input unit 7, the magnetic field setting unit 48 causes the magnetic field generator 2 to generate a peak magnetic field so that the magnetic field gradient of the peak magnetic field becomes the vertical magnetic field gradient set by the first magnetic field gradient setting units 46, 246, 246a, 346, and 446 at the position of the capsule endoscope 10, 210, 210b, 210c, and 210e. As a result, as illustrated in FIG. 43, differences between sparse and dense of the isodynamic lines Le1 to Le5 are adjusted so that the vertical magnetic field gradient of the peak magnetic field becomes the vertical magnetic field gradient that is obtained and set by the first magnetic field gradient setting units 46, 246, 246a, 346, and 446 at the position of the capsule endoscope 10. Then a vertical magnetic attracting force Fmp1 which makes a resultant force of the buoyancy Fb of the capsule endoscope 10 and the gravity force Fg and the vertical magnetic attracting force of the capsule endoscope 10 in the liquid W substantially zero is applied to the capsule endoscope 10 along with the buoyancy Fb of the capsule endoscope 10 and the gravity force Fg of the capsule endoscope 10. Therefore, the capsule endoscope 10 is kept in a substantially static state in the liquid W by the magnetic attracting force Fmp1.

When there is an input of the guidance instruction information for changing the position of the capsule endoscope 10 by the operation input unit 7, as illustrated in FIG. 44, the magnetic field setting unit 48 moves the magnetic field generator 2a relatively to the capsule endoscope 10 by an instructed amount of movement in the horizontal direction as illustrated by the arrow Y5 for the movement in the horizontal direction in the guidance instruction information for changing the position of the capsule endoscope 10. As a result, the peak position of the peak magnetic field also moves, and for example, a rightward magnetic attracting force Fmpv2 is applied to the capsule endoscope 10.

For the movement in the vertical direction in the guidance instruction information for changing the position of the capsule endoscope 10, the second magnetic field gradient setting units 47, 447, and 547 obtain a vertical magnetic field gradient of the peak magnetic field which generates a magnetic attracting force corresponding to the movement in the vertical direction and sets the obtained vertical magnetic field gradient as the second magnetic field gradient. The magnetic field setting unit 48 performs the guidance magnetic field setting process for setting a magnetic field having a vertical magnetic field gradient obtained by adding the second magnetic field gradient which is a vertical magnetic field gradient set by the second magnetic field gradient setting units 47, 447, and 547 to the first magnetic field gradient which is a vertical magnetic field gradient set by the first magnetic field gradient setting units 46, 246, 246a, 346, and 446 as the guidance magnetic field for the capsule endoscope 10. The magnetic field setting unit 48 causes the magnetic field generator 2 to generate a peak magnetic field so that the vertical magnetic field gradient of the peak magnetic field corresponds to the set guidance magnetic field at the position of the capsule endoscope. As a result, as illustrated in FIG. 43, differences between sparse and dense of the isodynamic lines Le1 to Le5 are adjusted so that the vertical magnetic field gradient of the peak magnetic field becomes the vertical magnetic field gradient corresponding to the set guidance magnetic field at the position of the capsule endoscope 10. Thereby, as illustrated in FIG. 44, for example, a vertically upward magnetic attracting force Fmph2 is applied to the capsule endoscope 10, and the capsule endoscope 10 is guided from a position P7 to a position P8 at upper light of the position P7 in accordance with the instruction of the guidance instruction information for changing the position of the capsule endoscope 10.

As described above, the first to the fifth embodiments can also be applied when the magnetic field generator 2 generates the peak magnetic field instead of the uniform magnetic field. When the peak magnetic field is used, the peak position and the vertical magnetic field gradient of the peak magnetic field are adjusted, so that it is possible to keep the capsule endoscope in a static state when the magnetic guidance is not performed. When the magnetic guidance is performed, the magnetic field on which the guidance instruction information for changing the position of the capsule endoscope 10 is reflected is further applied and the magnetic guidance is performed, so that reflectiveness of an input into the operation input unit 7 to the magnetic guidance can be enhanced.

Also, when the peak magnetic field is used, the capsule endoscope is operated as illustrated in FIG. 12 according to the guidance operation of the operation input unit 7 illustrated in FIGS. 11(1) and 11(2). When the up button 64U or the down button 64B is pressed, a peak magnetic field having a gradient in an instructed direction in the vertical direction is generated by the magnetic field generator 2 so that a magnetic attracting force is generated along the vertical axis Az, so that the capsule endoscope 10 is guided as illustrated by the arrow Y15 or the arrow Y16. When the joystick 62 is tilted in the left-right direction or in the frontward direction, the peak position of the peak magnetic field is moved to a position corresponding to the tilt direction of the joystick 62 at a moving speed corresponding to the tilt operation of the joystick 62, so that the capsule endoscope 10 is guided as illustrated by the arrow Y13 or the arrow Y15.

When the peak magnetic field is used, a part of the magnetic field generator 2 may be formed by a bed 404 for supporting a patient who is a subject illustrated in FIG. 45 and the magnetic field generator 2a which generates the peak magnetic field on the central axis. In this case, the peak magnetic field having a peak at a desired position inside the subject is generated by changing the relative position between the bed 404 and the magnetic field generator 2a. As illustrated in FIG. 45, for example, the bed 404 can be moved horizontally in the Y axis direction on the absolute coordinate system as illustrated by the arrow Y62 and the magnetic field generator 2a can be moved horizontally in the X axis direction on the absolute coordinate system as illustrated by the arrow Y60. In this case, the peak magnetic field having a peak at a predetermined position on a horizontal surface is generated by moving the bed 404 and the magnetic field generator 2a to change the relative position between the bed 404 and the magnetic field generator 2a. When the bed 404 can be moved in the X axis direction on the absolute coordinate system as illustrated by the arrow Y61 in addition to the Y axis direction on the absolute coordinate system, the relative position between the bed 404 and the magnetic field generator 2a may be changed by moving only the bed 404. When the magnetic field generator 2a can be moved in the Y axis direction on the absolute coordinate system in addition to the X axis direction on the absolute coordinate system, of course, the relative position between the bed 404 and the magnetic field generator 2a may be changed by moving only the magnetic field generator 2a. The magnetic field controllers 45, 245, 245a, 345, 445, and 545 move the peak position of the peak magnetic field by moving the magnetic field generator 2a relatively to the bed 404 and guide the capsule endoscope in accordance with the horizontal direction of the guidance instruction information for changing the position of the capsule endoscope 10, which is inputted from the operation input unit 7.

The magnetic field generator 2a generates the guidance magnetic field by, for example, a magnetic field generator realized by three-dimensionally combining three axis coils that generate a magnetic field in each axis direction on the absolute coordinate system. FIG. 46 is a schematic diagram illustrating the magnetic field generator illustrated in FIG. 45. As illustrated by a magnetic field generator 21 illustrated in FIG. 46, the magnetic field generator 2a is realized by three-dimensionally combining an X axis coil 21x that generates a magnetic field in the X axis direction on the absolute coordinate system, a Y axis coil 21y that generates a magnetic field in the Y axis direction on the absolute coordinate system, and a Z axis coil 21z that generates a magnetic field in the Z axis direction on the absolute coordinate system. The X axis coil 21x and the Y axis coil 21y are wrapped around an iron core 22 in a manner perpendicular to each other. The Z axis coil 21z is disposed above the X axis coil 21x and the Y axis coil 21y. The magnetic field controllers 45, 245, 245a, 345, 445, and 545 adjust differences between sparse and dense of the isodynamic lines by adjusting the amount of current flowing through the coils and adjust the vertical magnetic field gradient of the peak magnetic field.

Although, in the first to the fifth embodiments, examples in which the capsule endoscope 10 having a plurality of imaging units is used are described, of course, a monocular capsule endoscope having only the imaging unit 11A may be used.

Although, in the first to the fifth embodiments, the capsule endoscope 10 in which the permanent magnet 19 is used is described as an example, of course, it is not limited to this, and a capsule endoscope including an electromagnet instead of the permanent magnet 19 may be used.

In the second and the third embodiments, when determining whether or not there is the guidance instruction information for changing the position of the capsule endoscope 210 (step S24, S24-1, and S24-2), it is possible to determine only whether or not there is the guidance instruction information including a vertical direction component.

Thereby, when there is no guidance instruction information including a vertical direction component, the position of the capsule endoscope 210 in the vertical direction is maintained by the first gradient magnetic field on the basis of the detection result of the vertical direction movement detectors 249, 249b, 249c, 249d, and 249e and the position detector 249a, so that, even when the guidance instruction information including only a horizontal direction component is inputted, the position of the capsule endoscope 210 in the vertical direction is maintained by controlling the first gradient magnetic field. Thereby, the operability is improved.

Although, in the second and the third embodiments, the first gradient magnetic field is set on the basis of the physical parameter of the capsule endoscope 210, the physical parameter of the liquid, and the detection result of the vertical direction movement detectors 249, 249b, 249c, 249d, and 249e and the position detector 249a, it is not limited to this, and the first gradient magnetic field may be set on the basis of only the detection result of the vertical direction movement detectors 249, 249b, 249c, 249d, and 249e and the position detector 249a.

Thereby, even when the physical parameter of the capsule endoscope 210 and the physical parameter of the liquid are changed by the examination, a stable guidance performance can be realized without inputting each value, so that an operation to input each value is not required. Therefore, the operability is improved.

### EXPLANATIONS OF LETTERS OR NUMERALS

1, 201, 201a to 201e, 301, 401, 501 capsule medical device guidance system
2 magnetic field generator
3 transmitting/receiving unit
4, 204, 204a to 204e, 304, 404, 504 control unit
5 display unit
6 input unit
7, 7a operation input unit
8 storage unit
10, 210, 210b, 210c, 210e capsule endoscope
11A, 11B imaging unit
12 capsule-shaped casing
13A, 13B capsule-shaped casing
14A, 14B illumination unit
15A, 15B imaging element
16 wireless communication unit
16a antenna
17 control unit
18 power source unit
19 permanent magnet
41 image receiving unit
42 image display controller
45, 245, 245a, 345, 445, 545 magnetic field controller
46, 246, 346, 446 first magnetic field gradient setting unit
47, 447, 547 second magnetic field gradient setting unit
48 magnetic field setting unit
202, 202d magnetic field detector
202b, 202c position detection magnetic field generation unit
220 coil
220b magnetic sensor
220c LC marker
220e acceleration sensor
249, 249b to 249e vertical direction movement detector
249a position detector
306 first adjustment unit
367 slide bar

## Claims

1. A capsule medical device guidance system (1) for guiding a capsule medical device (10) which has a magnetic field response unit and is introduced into a liquid in a subject, the capsule medical device guidance system (1) comprising:
a magnetic field generator (2) configured to apply a magnetic field having a magnetic field gradient to the magnetic field response unit and to guide the capsule medical device (10);
a first setting unit (46) configured to set a first magnetic field gradient of a first magnetic field for generating a vertical magnetic attracting force which makes a resultant force of a buoyancy of the capsule medical device and a gravity force and the vertical magnetic attracting force of the capsule medical device in the liquid substantially zero, wherein the first setting unit (46) is further configured to acquire a physical parameter of the capsule medical device (10) and a physical parameter of the liquid and to set the first magnetic field gradient on the basis of said physical parameter of the capsule medical device (10) and said physical parameter of the liquid;
an operation input unit (7) to which guidance instruction information for guiding the capsule medical device (10) by a second magnetic field and moving the capsule medical device (10) from a first position to a second position is inputted;
a second setting unit (47) configured to calculate and set a second magnetic field gradient of the second magnetic field for generating a magnetic attracting force corresponding to the guidance instruction information inputted by the operation input unit (7); and
a control unit (4) configured:
to control the magnetic field generator (2) to automatically apply the first magnetic field having the first magnetic field gradient set by the first setting unit,
to determine whether the guidance instruction information is inputted to the operation input unit,
to control the magnetic field generator (2) to continue applying the first magnetic field and keeping the capsule medical device (10) in a static state in the liquid, if the control unit (4) determines that the guidance instruction information is not inputted to the operation input unit (7), and
to control the magnetic field generator (2) to further apply the second magnetic field in addition to the first magnetic field to move the capsule medical device (10) from the first position to the second position, if the control unit (4) determines that the guidance instruction information is inputted by the operation input unit (7).

2. The capsule medical device guidance system according to claim 1, wherein
the physical parameter of the capsule medical device (10) includes a volume, a mass, and a magnetic moment of the capsule medical device (10), and
the physical parameter of the liquid includes a density of the liquid.

3. The capsule medical device guidance system according to claim 2, further comprising:
a physical parameter related information input unit configured to input information related to at least one of the volume, the mass, and the magnetic moment of the capsule medical device (10) and the density of the liquid.

## Patentansprüche

1. Medizinische-Kapselvorrichtung-Führungssystem (1) zum Führen einer medizinischen Kapselvorrichtung (10), die eine Magnetfeldreaktionseinheit aufweist und in eine Flüssigkeit in einem Subjekt eingeführt ist, wobei das Medizinische-Kapselvorrichtung-Führungssystem (1) umfasst:
einen Magnetfelderzeuger (2), der dazu eingerichtet ist, ein Magnetfeld mit einem Magnetfeldgradienten auf die Magnetfeldreaktionseinheit anzuwenden und die medizinische Kapselvorrichtung (10) zu führen;
eine erste Einstelleinheit (46), die dazu eingerichtet ist, einen ersten Magnetfeldgradienten eines ersten Magnetfelds zur Erzeugung einer vertikalen Magnetanziehungskraft einzustellen, der eine resultierende Kraft einer Auftriebskraft der medizinischen Kapselvorrichtung und einer Gravitationskraft und der vertikalen Magnetanziehungskraft der medizinischen Kapselvorrichtung in der Flüssigkeit im Wesentlichen zu Null macht, wobei die erste Einstelleinheit (46) ferner dazu eingerichtet ist, einen physikalischen Parameter der medizinischen Kapselvorrichtung (10) und einen physikalischen Parameter der Flüssigkeit zu erfassen und den ersten Magnetfeldgradienten auf Grundlage des physikalischen Parameters der medizinischen Kapselvorrichtung (10) und des physikalischen Parameters der Flüssigkeit einzustellen;
eine Betriebseingabeeinheit (7), an die Führungsanweisungsinformation zum Führen der medizinischen Kapselvorrichtung (10) anhand eines zweiten Magnetfelds und Bewegen der medizinischen Kapselvorrichtung (10) von einer ersten Position zu einer zweiten Position eingegeben wird;
eine zweite Einstelleinheit (47), die dazu eingerichtet ist, einen zweiten Magnetfeldgradienten des zweiten Magnetfelds zur Erzeugung einer Magnetanziehungskraft entsprechend der anhand der Betriebseingabeeinheit (7) eingegebenen Führungsanweisungsinformation zu berechnen und einzustellen; und
eine Steuereinheit (4), die dazu eingerichtet ist:
den Magnetfelderzeuger (2) einzustellen, um das erste Magnetfeld mit dem anhand der ersten Einstelleinheit eingestellten ersten Magnetfeldgradienten automatisch anzuwenden,
zu bestimmen, ob die Führungsanweisungsinformation in die Betriebseingabeeinheit eingegeben ist,
den Magnetfelderzeuger (2) zu steuern, um das Anlegen des ersten Magnetfelds und das Halten der medizinischen Kapselvorrichtung (10) in einem statischen Zustand in der Flüssigkeit fortzusetzen, wenn die Steuereinheit (4) bestimmt, dass die Führungsanweisungsinformation nicht in die Betriebseingabeeinheit (7) eingegeben ist, und
den Magnetfelderzeuger (2) zu steuern, um ferner das zweiten Magnetfeld zusätzlich zu dem ersten Magnetfeld anzuwenden, um die medizinische Kapselvorrichtung (10) von der ersten Position zu der zweiten Position zu bewegen, wenn die Steuereinheit (4) bestimmt, dass die Führungsanweisungsinformation anhand der Betriebseingabeeinheit (7) eingegeben ist.

2. Medizinische-Kapselvorrichtung-Führungssystem (1) nach Anspruch 1, wobei
der physikalische Parameter der medizinischen Kapselvorrichtung (10) ein Volumen, eine Masse und ein magnetisches Moment der medizinischen Kapselvorrichtung (10) aufweist, und
der physikalische Parameter der Flüssigkeit eine Dichte der Flüssigkeit aufweist.

3. Medizinische-Kapselvorrichtung-Führungssystem (1) nach Anspruch 2, ferner umfassend:
eine Physikalischer-Parameter-bezogene-Informationseingabe-Einheit, die dazu eingerichtet ist, Information, die das Volumen, die Masse und/oder das magnetische Moment der medizinischen Kapselvorrichtung (10) und/oder die Dichte der Flüssigkeit betreffen, einzugeben.

## Revendications

1. Système (1) de guidage de dispositif médical de type capsule pour guider un dispositif (10) médical de type capsule qui a une unité de réponse à un champ magnétique et est introduit dans un liquide dans un sujet, le système (1) de guidage de dispositif médical de type capsule comprenant :
un générateur (2) de champ magnétique configuré pour appliquer un champ magnétique ayant un gradient de champ magnétique à l'unité de réponse à un champ magnétique et pour guider le dispositif (10) médical de type capsule ;
une première unité (46) de paramétrage configurée pour paramétrer un gradient de premier champ magnétique d'un premier champ magnétique pour générer une force d'attraction magnétique verticale qui met une force résultante d'une flottaison du dispositif médical de type capsule et d'une force de gravité et la force d'attraction magnétique verticale du dispositif médical de type capsule dans le liquide à sensiblement zéro, dans lequel la première unité (46) de paramétrage est en outre configurée pour acquérir un paramètre physique du dispositif (10) médical de type capsule et un paramètre physique du liquide et pour paramétrer le gradient de premier champ magnétique sur la base dudit paramètre physique du dispositif (10) médical de type capsule et dudit paramètre physique du liquide ;
une unité (7) d'entrée d'opération dans laquelle une information d'instruction de guidage pour guider le dispositif (10) médical de type capsule par un deuxième champ magnétique et déplacer le dispositif (10) médical de type capsule d'une première position jusqu'à une deuxième position est entrée ;
une deuxième unité (47) de paramétrage configurée pour calculer et paramétrer un gradient de deuxième champ magnétique du deuxième champ magnétique pour générer une force d'attraction magnétique correspondant à l'information d'instruction de guidage entrée par l'unité (7) d'entrée d'opération ; et
une unité (4) de commande configurée :
pour commander le générateur (2) de champ magnétique pour appliquer automatiquement le premier champ magnétique ayant le gradient de premier champ magnétique paramétré par la première unité de paramétrage,
pour déterminer si l'information d'instruction de guidage est ou non entrée dans l'unité d'entrée d'opération,
pour commander le générateur (2) de champ magnétique pour continuer à appliquer le premier champ magnétique et maintenir le dispositif (10) médical de type capsule dans un état statique dans le liquide, si l'unité (4) de commande détermine que l'information d'instruction de guidage n'est pas entrée dans l'unité (7) d'entrée d'opération, et
pour commander le générateur (2) de champ magnétique pour appliquer en outre le deuxième champ magnétique en plus du premier champ magnétique pour déplacer le dispositif (10) médical de type capsule de la première position jusqu'à la deuxième position, si l'unité (4) de commande détermine que l'information d'instruction de guidage est entrée par l'unité (7) d'entrée d'opération.

2. Système de guidage de dispositif médical de type capsule selon la revendication 1, dans lequel
le paramètre physique du dispositif (10) médical de type capsule inclut un volume, une masse, et un moment magnétique du dispositif (10) médical de type capsule, et
le paramètre physique du liquide inclut une densité du liquide.

3. Système de guidage de dispositif médical de type capsule selon la revendication 2, comprenant en outre :
une unité d'entrée d'information se rapportant à des paramètres physiques configurée pour entrer une information se rapportant à au moins un parmi le volume, la masse et le moment magnétique du dispositif (10) médical de type capsule et la densité du liquide.
